# EUROPEAN PATENT SPECIFICATION

(11) **EP 2 621 457 B1**
(45) Date of publication and mention of the grant of the patent: **23.08.2017**
(21) Application number: 11767600.7
(22) Date of filing: 29.09.2011
(51) Int. Cl.: A61Q 11/00, A61K 8/19, A61K 8/20, A61K 8/34, A61K 8/40, A61K 8/44, A61K 8/86, A61K 8/39, A61K 8/41, A61K 8/02, A61K 8/22

(54) **OXIDATIVE STABLE MICELLAR COMPOSITIONS**
HALTBARE OXIDIERENDE ZUSAMMENSETZUNGEN ENTHALTEND MICELLEN
COMPOSITIONS MICELLAIRES OXYDANTES STABLES

(30) Priority: 01.10.2010 WO PCT/CH2010/000240
(43) Date of publication of application: 07.08.2013
(73) Proprietor: Swissdent Cosmetics AG, 8001 Zürich (CH)
(72) Inventor: GHOSH, Sankha, Dublin 4 (IE); VELKOBORSKY, Vaclav, CH-8003 Zürich (CH)
(74) Representative: Grimm, Siegfried
(86) International application number: PCT/CH2011/000230
(87) International publication number: WO 2012/051727

(56) References cited:
- EP-A1- 2 047 889
- US-A1- 2007 053 849
- US-B1- 6 348 187
- R. NAGARAJAN: "Molecular Theory for Mixed Micelles", LANGMUIR, vol. 1, 1985, pages 331-341,

## Description

The invention relates to micellar compositions comprising two types of surfactant and a co-surfactant in the micellar phase and an oxidizing agent and a buffer in the aqueous phase. The invention further relates products containing such compositions, to the manufacture and use of such compositions and products.

It is known that oral malodor, plaque, gingivitis, periodontitis, carious lesion, hypersensitivity, discoloration of teeth, tongue coating and formation of tonsilloliths are known factors that significantly affect oral hygiene of a great amount of population around the world. It is also known that skin-care, hair-care and household hygiene are important factors that influence today's population and affect their welfare. It is also accepted that there exists an ever-existing need for providing improved pharmaceutical compositions.

Further, it is known that chlorine dioxide, oxygen and chlorine are excellent choice due to their profound role as selective anti-microbial agent at very low concentration. They function as bactericidal, bacteristatic, fungicidal, fungistatic, antimicrobial, antiseptic and antiviral as well as oxidizing agents (especially towards volatile sulfur compounds ("VSC's")) even at very low concentrations, making them efficient and suitable agent towards maintaining oral hygiene, skin hygiene, hair care, house hygiene. Further, they find applications pharmaceutical applications like treating and preventing viral, bacterial, fungal, microbial and protozoal infection related diseases and disorders. Precursors of them, which are capable of liberating/producing them via different means, are in general the ones that are attempted to formulate the compositions with. However, these precursors, due to their strong oxidizing property, underpin great amount of difficulty and challenge towards formulating commercial end products. There is a particular need for aqueous end products containing such precursors, that possess (1) reasonable shelf-life (preferably more than 24 months), (2) stability of the ingredients used over the shelf-life, (3) stability of the composition over the shelf-life, (4) retention of quality of flavor, fragrance and color, and (5) retention of packaging material. Consequently, there have been a number of attempts disclosed in the art.

It is further known that the presence of mono-alcohols, particularly ethanol, is disadvantageous in compositions or formulations for human use, e.g. due to redox reactions and / or desiccation. Desiccation, for example, promotes oral-malodor. Oxidation, for example, influences the stability of micellar compositions and constituents comprised therein.

It is further known that the presence of certain surfactants, such as surfactants from the group of dialkylethers, are disadvantageous in compositions or formulations for human use, e.g. due to limited oxidation stability.

It is further known that the presence of parabenes is disadvantageous in compositions or formulations for human use, particularly for sensitive users.

US 2008/0247972 discloses alcohol-free aqueous compositions comprising non-ionic surfactants, lipophilic ingredients oxidizing agents and buffers and its use as mouthwash for prevention and treatment of halitosis. It is suggested to use polysorbate 20, propylene glycol, PEG and polyoxyl with use level between 0.05 and 2% as surfactant. This document is silent about the use of ionic surfactants and co-surfactants. The compositions provided show low stability.

US 2003/0129144 discloses various single and dual system compositions comprising non-ionic surfactants, lipophilic ingredients, oxidizing agents and buffers and its use as mouthwash. This document is silent about the use of ionic surfactants and about the use of co-surfactants. The compositions provided show low stability.

US 2002/0197215 discloses compositions comprising non-ionic surfactants, lipophilic ingredients, oxidizing agents and butters and its use for oral care. This document is silent about the use of ionic surfactants, it rather stresses that the surfactants identified therein are an optimum, alternatives provide poor results. It was found that none of the disclosed examples survived for more than 3 weeks of stability test at 40C, resulting (1) phase separation, (2) yellowing and (3) depletion in NaClO2 concentration by more than 90%. These may be ascribed by the fact that the micellar aggregates were not (1) stable enough towards leaking entrapped flavor/lipophilic substance from the micellar core/interior, (2) micellar aggregates were not efficient enough to avoid confrontation between flavor phase and aqueous phase containing NaClO2, and (3) micellar aggregates may be too large to coalesce within a short time during storage releasing entrapped flavoring agents to confront with aqueous NaClO2 and stipulating phase separation to the overall composition. Therefore the disclosed compositions in this prior art document are not stable and suitable in formulating practical and commercial oral-care products.

DE4411557 discloses water in oil microemulsions comprising a combination of surfactants, whereby a surfactant from the group of dialkylethers is mandatory. The emulsions disclosed do not contain oxidizing agents. Aliphatic ethers, such dialkyl ethers, are known to be highly susceptible toward oxidation. Therefore, the disclosed dialkyl-ether containing micelles are not stable when the aqueous phase additionally contains an oxidizing agent. The products due to such oxidation reactions are harmful. Therefore it is not possible to formulate safe end-user commercial compositions containing oxidizing agents with a reasonable shelf-life and stability.

US 2004/0115159 discloses oil in water nano-emulsions comprising a combination of surfactants. The emulsions disclosed do not contain oxidizing agents. This is little surprising, as the constituents used in this document are sensitive towards oxidation.

US2007/0053849 discloses oral care compositions containing anti-bacterial and anti-inflammatory agents to prevent and treat bacteria-induced oral diseases and disorders. The specific compositions of example I contain a non-ionic surfactant but do not contain an ionic surfactant; they are not stable. Disclosed examples IIA-F do not contain non-ionic surfactant. This document discloses combinations of various active ingredients to achieve a certain therapeutic effect but is silent about combinations different surfactant classes.

US 5104644 discloses oral-solutions, mainly suitable as mouth-rinse/wash, containing hydrogen peroxides and ethanol. The compositions disclosed in this document are not stable. Further, this document is silent about combination of different classes of surfactants.

EP2047889 discloses tooth-whitening composition comprising of a whitening agent, a hydrophilic phase containing water, a hydrophobic phase containing oils, a surfactant (anionic or nonionic or amphoteric or zwitterionic) and a co-surfactant. The compositions disclosed in this document are not stable. Further, the document is silent about compositions comprising a combination of two different classes of surfactants.

US4431631 discloses compositions comprising hydrogen peroxide, ethanol, non-ionic surfactants and polyhydric alcohols. The compositions disclosed in this document are not stable. Further, the document is silent about compositions comprising a combination of two different classes of surfactants.

US6348187 discloses 2-component oral care, compositions in which part-1 contains micellar phase containing flavor, color, etc., lipophilic components while part-2 contains aqueous hydrogen peroxide. Thus, to enhance stability, a 2-component system was chosen, which is mixed prior to use.

Such compositions are considered disadvantageous for the tne-user. Further, the document is silent about compositions comprising a combination of two different classes of surfactants.

Consequently, it is an object of the present invention to provide improved compositions and to mitigate at least some of the above drawbacks.

These objectives are achieved by a composition as defined in claim 1. Further aspects of the invention are disclosed in the specification and independent claims, preferred embodiments are disclosed in the specification and the dependent claims.

The present invention will be described in more detail below. It is understood that the various embodiments, preferences and ranges may be combined at will. Further, depending on the specific embodiment, selected definitions, embodiments or ranges may not apply.

Unless otherwise stated, the following definitions shall apply in this specification:

The term "containing" shall, in the context of this invention, also include the meaning of comprising or consisting of.

The term "wt.-%" denotes the weight of a specific substituent in relation to the inventive composition, unless stated otherwise.

The term "treatment of a disorder" shall, in the context of this invention, also include the prevention and/or delay of progression of said disorder.

The term "stable" is known in the field. It particularly denotes, the fact that a certain parameter remains unchanged or essentially unchanged during the intended use of the compositions / end-products described herein. For examples, micelles are "stable" if size and size distribution remain constant within a range of +/-10% during storage and use. Further, constituents are stable towards oxidation or pH change if 10% or less of said constituent are decomposed during storage and use.

The term "Micelle" is known in the field. It particularly denotes an aggregate / shelf-assembly (Mₙ) of surfactant molecules (nM) dispersed in a liquid colloid, where M denotes a surfactant molecule, often referred to as surfactant monomer, Mₙ denotes a micelle due to aggregation of n surfactant monomers and n is number of surfactant molecule/monomers. It is also known that there are two types of micelles: (1) Direct / typical micelle, designated by o/w, and (2) reverse / inverse micelle, denoted by w/o. It is generally accepted that micelles are not static entities; this is reflected by the known term "micellar migration". Micellar migration is known to occur through three mechanisms, viz. 1. Intermicellar migration, 2. Intermicellar collision and 3. Micellar fragment migration. Intermicellar migration involves (1) exit of surfactant monomers/molecules and solubilized ingredients from one micelle, followed by (2) diffusion in continuous medium / solvent, and (3) association to other micelle. Micellar migration involving intermicellar collision occurs via (1) temporary coalescence of two micelles (fusion of two micelles/micellar fusion) during which exchange of surfactant monomers, solubilized additives and micellar fragments between the merged micelles take place, followed by (2) separation (micellar fission) and (3) re-formation of two micelles. Micellar fragment migration occurs through (1) fragmentation of a micelle and loss of a micellar fragment that contains surfactant monomers, solubilized additives and co-surfactant molecules, followed by (2) diffusion in continuous phase / solvent and (3) coagulation/association to other micelle. From the above it is apparent that the effects of micellar migration are often non-desired.
A plurality of micelles in a composition is considered the "micellar phase".

A "micellar composition" is any composition containing micelles, thus including liquid, semi-solid and solid compositions.
The term "Co-surfactant" is known in the field. It particularly denotes the substance that collaborates with surfactants in reducing interfacial tension, but having no surfactant properties itself. Co-surfactants are typically present in the micellar and the aqueous phase.
The term "liquid oral solution composition" is known in the field. It particularly denotes a mouthwash, mouth spray, gurgling rinse and mouth rinse.
The terms "emulsion" and "microemulsion" (collectively: (micro)-emulsion) are known in the field. They particularly denote a liquid colloid in which one or a mixture of liquid components (dispersed phase) is dispersed in other liquid continuous phase (solvent), comprised of one or a mixture of liquids, often in a combination of a third component, an emulsifier, having surfactant property that enhances the kinetic stability of the emulsion through lowering the interfacial tensions due to formation of micellar aggregates.
A microemulsion is a thermodynamically stable isotropic dispersion of two immiscible liquids stabilized by surfactant, often in combination with a co-surfactant. Microemulsions typically comprise of homogeneously distributed droplets with a diameter in the range of more than 0.1 and less than 100 micrometers, preferably 0.4-20 micrometers ("micellar phase", measured by laser diffraction) throughout the continuous medium making it clear and transparent or opalescent. Although the microemulsions appear to be a clear transparent/opalescent mono-phase solution to human eye, they are actually a ternary system, designated as oil/surfactant/water or water/surfactant/oil, wherein surfactant molecules form the interface through self-assembly with various possible structures, of which micellar assembly is most frequent and common.

An emulsion is an isotropic dispersion of two immiscible liquids stabilized by surfactant, often in combination with a co-surfactant. Emulsions typically comprise of homogeneously distributed droplets with a diameter in the range of 1 - 100 micrometers, preferably 1-20 micrometers ("micellar phase", measured by laser diffraction) throughout the continuous medium making it turbid.
For (micro)-emulsions, the aqueous phase may contain dissolved electrolytes and any water-soluble spices, while the oil phase may be a mixture of water-insoluble lipophilic substances.
There are two types of (micro)-emulsions of which one is "direct" (o/w) wherein the oil, which is the minor componet, is dispersed in water, which is the major component. The second type is "reverse" ("inverse" w/o) wherein water, which is the minor component, is dispersed in the oil, which is the major component.

The term, "gel", is known in the field. It particularly denotes a semi-solid colloid which contains both solid and liquid components. The solid component, which is called "gelling agent", comprises a three-dimensional network of interconnected molecules or aggregates which immobilize the liquid continuous phase. Hydrogels have an aqueous phase/medium and organogels have an organic solvent as the liquid continuous phase/medium. Gels may also be classified based on the nature of the bonds involved in the three-dimensional gel-network. In chemical gels, strong covalent bonds comprise the gel network, and in physical gels, the hydrogen bonds, electrostatic and van der Waals interactions, which are relatively weaker. The liquid continuous medium/phase in emulsions and microemulsions may be immobilized by introducing a suitable gelling agent to produce emulsion gels and microemulsion gels. Gels are capable of "suspending" solid particles over the entire volume due to visco-elastic and yield-point/value properties.

The term "end product" particularly denotes a product designated to the end user, which contains a micellar composition as defined herein. Such end product may be in the form of an oral care composition, skin care composition, hair care composition, pharmaceutical composition, or household care composition.
In a **first aspect,** the invention relates to a micellar composition free of ethanol, containing (i.e. comprising or consisting of) a micellar phase and a continuous aqueous phase; wherein the micellar phase comprises (1) one or more ionic surfactants, (2) one or more non-ionic surfactants, (3) one or more co-surfactants selected from the group consisting of polyols, (4) optionally one or more lipophilic ingredients, and wherein the continuous phase comprises (5) one or more oxidizing agents, (6) one or more buffers, (7) optionally one or more hydrophilic ingredients, (8) optionally one or more excipients. The amount of (3) is 5 - 30 wt-% of the composition. The ratio of (1):(2) is in the range of 1:1.2 to 1:20, preferably 1:1.25 to 1:15, much preferably 1:1.5 to 1:10. The micelles of said micellar phase are mixed micelles and have an average diameter of more than 0.1 and less than 100 micrometers (determined by laser diffraction).
A novel approach based on a stable micellar carrier of lipophilic substances in o/w colloids, wherein two phases are precisely designed to avoid mutual confrontation over a long period of time, sufficient to be pertinent as commercial end products is provided. It was surprisingly found that the inventive compositions have a shelf-life of 36 months or longer at room temperature, do not suffer from yellowing discoloration, do not suffer from denaturation of flavor and taste and do not develop undesirable chlorine odor. Further, the inventive compositions retain at least 80% of oxidizing agent at the length of 36 months. Further, the inventive compositions show a pronounced effect on oral hygiene and / or oral health, particularly provide excellent results in controlling and/or reducing halitosis. Halitosis (Oral malodor) is attributable to systemic disorders including oral carcinomas, diabetes, liver and kidney abnormalities, medications which change the oral environment, ENT problems such as chronic sinusitis, tonsillitis and inflamed adenoids, as well as gingivitis and periodontal diseases. Without being bound to theory, it is believed that the inventive composition does not only P105387EP01 desc 2016-12.docx control but reduce VSC instantly by oxidation and eliminate the source of VSC by killing the bacteria that produce VSC.

It was found that a suitable mixture of ionic surfactant(s), non-ionic surfactant(s) and co-surfactant(s) results in formation of advantageous "mixed micelles" with compact interface. Such micelles are (1) optimal in size towards efficiently accommodating lipophilic additives in the micellar interior/core, (2) thermodynamically and kinetically more stable, (3) resistant towards micellar fragmentation and collision migrations and (4) capable of shielding the lipophilic core and dissolved lipophilic substances therein from the aqueous phases as to circumvent intermicellar migrations and therefore enhancing the stability of ingredients.

The invention is explained in further details below:
**Constituents:** It is understood that each constituent (1) to (8) may be present as one single component (e.g. a single ingredient) or as a combination of components (e.g. two or more ingredients). As outlined below, it was found particularly suitable to employ more than one, e.g. two or three, buffers in the inventive composition. The constituents of the inventive composition are known per se, i.e. commercially available or available by known methods, although not combined as disclosed herein.

**Constituent (1):** Ionic surfactants are known in the field. Suitable ionic surfactants are selected from the group consisting of zwitterionic, anionic and cationic families of surfactants. The selection preference depends inter alia on the type of the desired compositions, compatibilities with the ingredients of the composition, pH of the composition. Suitable amount depend inter alia on the type of the desired composition in which the formulation is to be incorporated. For example, anionic surfactants are preferred over the cationic surfactants for alkaline end products, and the latter is preferred over the former for acidic end products, while zwitterionic surfactants can be used for both as well as for pH neutral end products.

Preferred zwitterionic surfactants are selected from the group consisting of Betaine family (such as Cetyl Betaine, Cocamidopropyl betaine, Dodecyl betaine, LAURAMIDOPROPYL Betaine), Amino Betaine family (such as glycine betaines), Betaine Oxide family (such as Coconut betaine oxide), Amine N-oxide family (such as Lauraime oxide, Cocamine oxide, Lauramidopropylamine oxide, Cocamidopropylamine oxide, Palmitamine Oxide, Decylamine oxide). Particularly preferred zwitterionic surfactants are selected from the group consisting of Lauramine Oxide (e.g. OXIDET DM-20), Cocamine Oxide (e.g. OXIDET DM-246, OXIDET DMCLD), Myristamine oxide (e. g. OXIDET DM-4) and Cocamidopropylamine oxide (e. g. OXIDET L-75 C); all obtainable from KAO Chemicals, Spain.

Preferred anionic surfactants are selected from the group consisting of Carboxlylate family (such as Sodium Laureth-11 Carboxylate, Sodium Capryleth-9 carboxylate, Sodium Buteth-2 carboxylate, SODIUM PEG-7 OLIVE OIL CARBOXYLATE), Sulfate family (such as Sodium Hexylethyl Sulfate, Sodium Lauryl Sulfate, Sodium Laureth Sulfate), Sarcosinate family (such as Sodium Lauroly Sarcosinate, Sodium Laureth Sulfosarcosinate), Sulfonate family such as (DISODIUM COCAMIDO PEG-3 SULFOSUCCINATE, DISODIUM COCOAMPHOCARBOXY-ETHYL-HYDROXYPROPYL SULFONATE), Sulfosuccinate family (such as DISODIUM STEARYL SULFOSUCCINATE, DISODIUM C12-14 PARETH-2-SULFOSUCCINATE, DISODIUM CETEARYL SULFOSUCCINATE, POTASSIUM CUMENESULFONATE), Phosphate family (such as PEG-26-PPG-30 PHOSPHATE, TRISODIUM LAUROAMPHO PG-ACETATE CHLORIDE PHOSPHATE, Alkyl Phosphates, C12-13 PARETH-10 PHOSPHATE, CETETH-20 PHOSPHATE, POTASSIUM DIHYDROXYETHYL COCAMINE OXIDE PHOSPHATE, SODIUM LAUROAMPHO PG-ACETATE PHOSPHATE), Acetate family (such as SODIUM METHOXY PPG-2 ACETATE, SODIUM OLEOAMPHOACETATE). Particularly preferred anionic surfactants are selected from the group consisting of Laureth-11 Carboxylic acid (e.g. AKYPO RLM 100), Sodium Laureth-11 Carboxylate (e.g. AKYPO SOFT 100 NV, AKYPO SOFT 100 BVC), Laureth-6 Carboxylic acid (e.g. AKYPO RLM 100 CA), Laureth-8 Carboxylic acid (e.g. AKYPO RLM 70), Sodium Laureth-8 Carboxylate (e.g. AKYPO SOFT 70 NV), Sodium Laureth-6 Carboxylate (e.g. AKYPO SOFT 45 NV), Capryleth-9 Carboxylic acid (e.g. AKYPO LF 2), Capryleth-6 Carboxylic acid (e. g. AKYPO LF 1) and Capryleth-9 carboxylic acid/ Buteth-2 carboxylic acid (e. g. AKYPO LF 6), all of them obtainable from KAO Chemicals, Germany.

Preferred cationic surfactants are selected from the group consisting of quaternary ammonium salts, Benzalkonium chloride, Cetylpyridinium chloride, Cetylpyridinium bromide.

**Constituent (2):** Non-ionic surfactants are known in the field. The selection preference depends inter alia on the type of the desired compositions, compatibilities with the ingredients of the composition, pH of the composition. Suitable amount depends inter alia on the type of the desired composition in which the formulation is to be incorporated. Depending on o/w vs. w/o nature of the end product, the non-ionic surfactant or a group of them are so chosen as to keep the mean HLB value in the range of 1-10 for w/o case while 10.1-20 for o/w case, respectively. The ratio between ionic and non-ionic surfactants (1:1.5-20) and the use level of co-surfactant typically remain unchanged, irrespective of the polarity of the end product. Preferred non-ionic surfactants are selected from the group consisting of Sorbitan mono-, di- and tri- esters of fatty acids, their poly-ethyleneoxide (PEG) derivatives, polyethyleneglycol-polypropyleneglycol-polethyleneglycol block (PEG-PPG-PEG) polymer family, polypropylene-polyethylene-polypropylene (PPG-PEG-PPG) block polymer family, family of glyceryl esters of fatty acids, family of polyglyceryl esters of fatty acids, family of glycol esters of fatty acids, family of polyglycol esters of fatty acids, fatty alcohol family, family of fatty ether alcohols, family of PPG derivatives of fatty ether alcohols, family of PEG derivatives of esters of fatty ethers and fatty acids, family of fatty amines and their PEG and PPG derivatives, family of fatty amides and their PEG derivatives.

**Constituent (3):** Co-surfactants are known in the field. For the inventive compositions, poly-ol species are chosen. Preferred co-surfactants are selected from the group consisting of propylene glycols, glycerin, sorbitol, Polyethylene glycols, Polypropylene glycols.
As outlined above, the inventive composition may be present in the form of an emulsion (such as an o/w emulsion) or as a microemulsion (such as an o/w microemulsion). To obtain an emulsion, which is typically turbid, comparatively low amounts of co-surfactant, typically in the range of 5-15 wt-% are used. To obtain a microemulsion, which is typically clear, comparatively high amounts of co-surfactant, typically in the range of 15-30 wt-% are used. Thus, the invention also relates to a micellar composition as described herein (a) in the form of an emulsion with 5-15 wt-% co-surfactant or (b) in the form of a micro-emulsion with 15-30 wt-% co-surfactant.

**Constituent (4):** One or more lipophilic ingredients, such as flavoring oils, may be employed in the inventive composition. In principle, a wide range of lipophilic ingredients, such as flavoring, fragrance, perfume agents, may be formulated in the inventive composition (i.e. solubilised in the core of the inventive micelles). Favoring agents include natural and synthetic ones.
In one embodiment, flavoring oils are selected from the group consisting of Peppermint oil, Spearmint oil, Birch / Wintergreen oil, Eucalyptol, Anise oil, Orange oil, Lemon oil, Eugenol, Clove oil, Freshmint flavour, Sparkling mint flavour, Green apple flavour, Limonene, peppermint fragrance, spearmint fragrance, Lavender fragrance, jasmine fragrance, pineapple fragrance, various perfumes, menthol, menthane, anethole, methyl salicylate, cassia, 1-methyl acetate, sage, oxanone, alpha-irisone, marjoram, propenyl guaethol acetyl, cinnamon, vanillin, thymol, linalool, cinnamaldehyde, ethyl cinnamate, beta-caryophyllene, N-ethyl p-menthane-3-carboxamide, 3-1menthoxypropane-1,2-diol, menthone glycerol, benzyl nicotinate, benzocaine, lidocaine, clove bud oil, glycerol acetyl. In particular, spearmint flavor/fragrance and peppermint flavor/fragrance with cooling agent (effect) under tradenames Cool Spearmint C123 and Cool Peppermint W439, respectively, supplied by Huber the Nose, Switzerland, have proven promising.
In one further embodiment, flavoring oils are selected from the group consisting of Peppermint oil, Spearmint oil, Wintergreen oil, Eucalyptus oil. Particularly good results are obtained when said flavoring oils are extracted from leaves of the corresponding plants. Such oils are commercially available may be used as "extra pure" (e.g. supplied by Essencia, Winterthur, Switzerland).
Concentration of constituent (4) may vary over a broad range, typically 0.01-8 wt-%. Particularly suitable amounts of constituent (4) are in the range of 0.2 - 8 wt.-%, preferably 0.5 - 5 wt.-%.

**Constituent (5):** Oxidizing agents are known in the field. Suitable oxidizing agent include, (1) Chlorine Dioxide (ClO2) releasing agents, (2) Oxygen releasing agents, (3) Chlorine releasing agents and (4) Compounds with oxidation property. Non-limiting examples of ClO2 releasing agents include Alkali, Alkali Earth and Periodic table group 12 metal chlorites, chlorates and perchlorates. Non-limiting examples of oxygen releasing agents include Hydrogen peroxide, PVP-hydrogen peroxides, Carbamide/Urea Peroxide, Organic peroxides, Alkali metal, Alkali earth metal, and periodic table group 12 metal peroxides, percarbonates, perborates, persulfates, peroxyphosphates, oxoborates, perthalates. Non-limiting examples of Chlorine releasing agents include Alkali, Alkali earth and periodic table group 12 metal hypochlorites and chloro-hychlorites. Non-limiting examples of oxidizing compounds include metal nitrates and permanganates.
Precursors of ClO2, oxygen and chlorine are often pH-dependent; such pH-dependant precursors can be classified into three groups. Alkali pH stable group includes Alkali, Alkali earth and Periodic table group 12 metal salt type of precursors, such as NaClO2, KClO3, Ca(ClO2)2, NaOCl, Ca(OCl)2, Ca(OCl)Cl, CaO2, MgO2, ZnO2, etc. Acidic pH stable group includes H2O2, Carbamide/Urea peroxide, PVP-H2O2, etc. Neutral pH stable group includes organic peroxides, Alkali metal nitrates, etc.
Suitable amounts of constituent (5), if present, are in the range of 0.01 - 40 wt.-%, preferably 0.1 - 10 wt.-%.
In an advantageous embodiment, the present invention relates to micellar compositions and end-products, as described herein, which contains an oxidizing agent, typically within the aqueous phase. If the aqueous phase of the micellar phase (or of the end composition) contains an oxidizing agent, the preferred ionic surfactants, non-ionic surfactants and co-surfactant are the ones that possess fair stability towards oxidation. Such redox-stable surfactants / co-surfactants are known to the person skilled in the art. Consequently, in this embodiment, surfactants exclude dialkyl ethers and co-surfactants exclude monohydric-alcohols.

**Constituent (6):** pH buffering agents ("buffers") are known in the field. For the inventive compositions, one or more buffers (preferably two or three) may be employed. The choice of a specific buffer depends on the oxidizing agent used and may be selected by a person skilled in the art. Buffers may be added in order to maintain pH of a composition within the desired range over the shelf-life. Addition of one or multiple buffers is particularly important to the compositions that contain oxidizing agents in order to stabilized (improve the life) of certain classes of oxidizing agents, especially the pH-dependent precursors of ClO2, oxygen and chlorine, in the composition. Suitable buffers thus help to stabilize such precursors in a composition by precisely administering and fixing the pH over a certain desirable range. Further, pH buffering agents may function as pH regulator that may activate the oxidizing action (such as by releasing ClO2, oxygen, Chlorine) of the precursor upon application due to a change in pH of the medium.
By incorporating multiple pH buffer agents, it is possible to regulate the release of ClO2, oxygen and chlorine in a sustained fashion upon application, since overlap in pH buffer capacities gives rise to a wide range where pH change is hindered. For certain applications, sustained release is more desirable over rapid release, since the former is associated with higher bioavailability. Multiple buffers with tailored overlap in buffer capacities further improve the stability and life of such precursors. Therefore, pH buffering agents offer scope to manage stability of the said precursors, their mode of release and the overall stability as well as functionality of the compositions.
Any pH buffer agent known in the field can be used. Suitable buffers include borate (Alkali metal hydroxide/Boric acid) buffer, phosphate (Alkali metal salts of boric acids) buffer, carbonate/bicarbonate (alkali metal carbonates/bicarbonates) buffer, Alkali metal fluorophosphates/phosphoric acid buffer.
The amount of (6) may vary over a broad range, suitable amounts of constituent (4) are in the range of 0.01-20wt-%. The **pH** of the inventive composition may vary over a broad range and may be adjusted by suitably selecting the buffer(s). Appropriate pH depends on the intended use of the final product. Oral care compositions are preferably in the alkaline range; hair care and skin case are preferably in the acidic range while pharmaceutical products are preferably in the neutral range. In an advantageous embodiment, the invention thus relates to an oral care composition as described herein, having a pH between 7 and 11, preferably between 7.5 and 9.5, much preferred between 8 and 9. In a further advantageous embodiment, the invention thus relates to a hair / skin care composition as described herein, having a pH between 7 and 4, preferably between 6.5 and 5, much preferred between 6 and 5.5. In an advantageous embodiment, the invention thus relates to an pharmaceutical composition as described herein, having a pH between 6 and 8, preferably between 6.5 and 7.5, much preferred between 6.8 and 7.2.

**Constituent (7):** Optionally, one or more water soluble and / or hydrophilic ingredients may be employed in the inventive compositions. Such ingredients are known in the filed. Water soluble ingredients include, as a non-limiting example, emollients and isotonic agents.

**Constituent (8):** Excipients are known in the field and may be employed to tailor the inventive compositions. Excipients include, as a non-limiting example, binders, viscosity enhancers, abrasives.

The following components may be added to the inventive composition, to specifically adapt it to an intended use. These components may fall within the meaning of lipophilic ingredient (4), hydrophilic ingredient (7) or excipient (8). It is also known that components may serve multiple functions, e.g. the same compound may function as anti carious and anti-plaque agent or as detergent and thickening agent.

**Activators:** One or more activators may be added to the inventive composition. They provide further control in and facilitate production of Chlorine dioxide, Oxygen and Chlorine from their precursors as defined herein. Such activators may be delivered alone or as a separate composition of suitable physical form, such as liquid, semi-solid and solid, and such composition may optionally contain inventive micellar composition. Such activators are known in the field and may be classified as three main families comprising (1) pH modifier, (2) oxidizing agent and (3) reducing agent, while each family may comprise one or more inorganic or organic compounds or a combination thereof as member. Such activator compositions may be applied either simultaneously or sequentially with the inventive compositions. pH modifier class may have acidic or alkaline pH and may contain one or more acids, alkalis, acidic salts, alkaline salts and pH-buffer agents. Non-limiting examples of pH modifier based activators include organic acids such as Citric acid, Tartaric acid, acetic acid, ascorbic acid, etc., organic acid anhydrides such as maleic anhydride, inorganic acids such as Hydrochloric acid, boric acid, phosphoric acid, etc., inorganic acid salts such as monosodium dihydrogen phosphate, monopotassium dihydrogen phosphate, calcium chloride, magnesium chloride, magnesium sulfate, etc., organic acid salts such as urea phosphate, inorganic alkalies such as alkali, alkali earth and periodic table group 12 hydroxides and oxides, inorganic alkaline salts such as alkali metal di- and tri-basic phosphates, mono- and di-basic carbonates, metal borates, metal halophosphates, hypocholites, cholites, cholates, peroxides, etc., organic bases (alkali) such as amines, organic alkaline salts such as alkali, alkali earth and periodic table group 12 metal mono and poly carboxlates, organic phosphates, such as calcium glycerophosphate, sodium glycerophosphate, any acidic and alkaline buffer agents, etc. Non-limiting examples of oxidizer based activators are already given. Reducing agent based activators can be any organic or inorganic compounds with reducing property such as aldehyde, hydrides, aldehyde-carbohydrates, metal iodies, metal bromides, metal hypochlorites, etc. Redox type activators may be used alone or in a combination with a suitable pH modifier type.
Thus, the invention also relates to a micellar composition as described herein in the form of a kit of parts wherein the first part comprises the inventive composition as described above and the second part comprises one or more activators, or activator compositions.

**Anti-carious agents:** One or more anti-carious agents may be employed in the inventive composition. Anti-carious agents are known in the field; they prevent formation of carious lesion by re-mineralizing and reducing plaque. (re)-mineralizing and anti-plaque agents as defined above are also suitable as anti-carious agents.

**Anti-plaque agent:** One or more anti-plaque agent may be employed in the inventive composition. Anti-plaque agents are known in the field; they lower plaque count due to anti-bacterial, anti-fungal, anti-protozoal and/or anti-microbial properties. The oxidizing agent as defined herein may be used as an anti-plaque agent.
Other anti-plauqe agents,suitable for the inventive compositions, include natural substances (such as Xylitol, Thymol, Eucalyptol, Euganol, Oil of Clove, Sweet Birch Oil, Wintergreen Oil, D-Limonene, Mentha Paprita L oil, Spearmint oil) and synthetic substances (such as Methyl Paraben, Ethyl Paraben, Propyl Paraben, Domiphene Bromide, Salicylanide, Zinc ions, Stannous ions, Octenidine, Delmopione, Octapinol, Polyaminopropyl Biguanide).
Concentration may vary over a broad range, typically 0.01-10 wt-%.

**Anti-tartar agents:** One or more anti-tartar agents may be employed in the inventive composition. Anti-tartar agents are known in the field; they are the ones that prevent undesirable calcification of oral plaque. In oral care compositions, such anti-tartar agents are advantageously present. Suitable anti-tartar agents includes Alkali metal Pyrophosphates (such as Tetrasodium pyrophosphate, Tetrapotassium pyrophosphate, Disodium dihydrogen Pyrophospahtes, Dipotassium dihydrogen Pyrophosphates), 1-Azocycloheptane-2,2-diphosphonate, Ethane-1-hydroxy-1,1-diphosphonate, Alkali metal salts of Polyphosphate, Phosphocitrate salts, Polyacrylates, Polycarboxylates, Alkali Metal salts of EDTA, Zinc Chloride, Polyphosphonates, Polyepoxysuccinates, Nitrilotriacetic Acid and its salts of Alkali Metals, Ethylenediamintetraacetic acid (EDTA), Alkali Earth Metal Salts of Citrate etc. Alkali Pyrophosphates are the most preferred ones and reside in aqueous phase.
Concentration may vary over a broad range, typically 0.1-10 wt-%.

**Aqueous phase:** According to the invention, the aqueous phase contains constituents (5), (6), (7) dissolved and constituent (8) optionally dissolved, or as a solid phase. The water employed may be any purity grade of water compliant with the intended use as an oral care product. Advantageously, de-ionized water is used. Suitable amounts of water are in the range of 60.05 - 84.5 wt.-%, preferably 70 - 80 wt.-%.

**Colorants:** One or more coloring agents may be employed in the inventive composition. Colorants include a. Suspendable pigment agents, b. Oxidation stable water soluble inorganic and organic compounds and c. lipophilic chromophores.
The present invention offers an excellent and novel means to introduce color to a composition through loading lipophilic colorant molecules in the core of the inventive o/w micelles. This is very effective when the aqueous phase in o/w type compositions contains oxidizing agents, since the lipophilic colorants are protected from confronting aqueous phase. In w/o compositions, the water soluble/hydrophilic colorants can reside in the hydrophilic core/interior of the inventive micelles, as well, offering a new means to introduce color through inventive w/o micelles. The same holds true for flavoring / fragrance / perfumes and any other lipophilic ingredient.

**Detergents / foaming agents:** One or more foaming agents may be employed in the inventive composition, in addition to the surfactants described above. Suitable foaming agents may be selected from the group consisting of ionic surfactants and non-ionic surfactants. For oral care applications, mild detergents are particularly preferable. For hair and skin applications the use of mild detergents is also preferable, while for household applications (such as floor, window, any hard surface, etc.), detergent with hydrotropic property are preferred.
Concentration may vary over a broad range, typically 0.1-40 wt-%.

**Emollients:** One or more emollients may be employed in the inventive composition. Emollients are substances that soften and smooth the skin; they are often referred to as conditioning (skin conditioning, hair conditioning) agents. Suitible are broad range of compounds, both natural and synthetic, are known in the field to have emollient property. Suitable emollients may be selected from the group consisting of fatty alcohols, fatty acids, esters of fatty acids, metal salts of fatty acids, glycerides, fatty ether alcohols, fatty ether acids, fatty ether esters, fatty amides, wax, wax esters, hydrocarbons, glycols, glycerols, C10-50 Alkyl Methicones, Dimethicones, Extracts from plants/fruits/leaves, Coenzymes (Coenzyme A), vitamins (their derivatives), etc.
Concentration may vary over a broad range, typically 0.1-20 wt-%.

**Enzymes:** One or more enzymes may be employed in the inventive composition. Suitable enzymes include catalytically active protein substances within the class of hydrolases, which break down or hydrolyze proteins (proteases), carbohydrates (carbohydrases), fatty substances (lipases), or complexes of these types of substances. These enzymes and the crude enzyme preparations containing them are obtained from natural sources or by the action of microorganisms on an appropriate medium having a nitrogen source and a carbon source. For example the microorganism Bacillus subtilis when cultivated in a medium containing a protein, such as soybean meal, and glucose, produces a proteolytic enzyme suitable for use in accordance with the invention. One useful protease thus prepared has an activity of about 2.4 million units per gram at pH 7 when assayed against a casein substrate as described hereinafter. Non-limiting example of protease includes Alcalase (obtainable from Novo Industry A/S), Maxatase (obtainable from Pfizer), Protease AP-100 (obtainable from Monsanto), Prolase 300 (obtainable from Wallerstein), Trypsin derivable from bovine or porcine pancreas, Chymotrypsin derivable from bovine, chicken, or turkey pancreas, Papain, Ficin, Bromelin, M-Zyme, Rhozyme P-11 (obtainable from Rohm and Hass). Non-limiting example of carbohydrase enzymes includes, dextranase, cellulase, alpha-amylase, beta-amylase, Mylase 100 (obtainable from Wallerstein). Non-limiting examples of lipase includes, Lipase 4000, Lipase B, Lipase 448. Typically, enzymes are obtained in lyophilized form and thus they constitute group of lipophilic ingredients (4) which are solubilised in the core of the inventive micelles.
Concentration may vary over a broad range, typically 0.01-10 wt-%.

**Humectants:** One or more humectants may be employed in the inventive composition. Suitable humectants may be chosen from anhydrous or hydrous humectants. Anhydrous humectants are preferably selected from the group consisting of anhydrous glycerol, anhydrous Propylene Glycol, Polyethylene Glycols (PEG 400, PEG 600, PEG 800, etc.), Polypropylene glycols, hydrogenated vegetable oils, hydrogenated polybutene, hydrogenated polyisobutene, hydrogenated polydecene, hydrogenated polyisodecene , ethanol, mono and poly esters of fatty acids, fatty alcohols and mineral oils. Hydrous humectants are preferably selected from the group consisting of water, glycerol, Propylene glycol, PEG, PPG and D-sorbitol.
Concentration may vary over a broad range, typically 1-80% wt-%.

**Hyaluronic acid:** Hyaluronic acid (HA) may be employed in the inventive composition. HAs with molecular weight ranging between 25 and 4000 kDa and with various degrees of substitution and alkali metal salts thereof are known in the field for a range of applications in tissue engineering. The presence of hyaluronic acid in epithelial tissue has been shown to promote keratinocyte proliferation and increase the presence of retinoic acid, effecting skin hydration. It promotes collagen synthesis and normal skin function. Hyaluronic acid is critical to the structural integrity of the dermal collagen matrix. Hyaluronic acid plays crucial role in wound healing process. HA plays an important role in the normal epidermis. HA also has crucial functions in the reepithelization process due to several of its properties: it serves as an integral part of the extracellular matrix of basal keratinocytes, which are major constituents of epidermis; its free-radical scavenging function and its role in keratinocyte proliferation and migration. Further, HA and metal salts thereof, especially the ones with higher substitutions/cross-links, have gelling/thickening property, and often such compositions impart excellent skin-feeling. Due to these advantageous functions, HA, and salts thereof, may be incorporated in skin care, oral care, topical pharmaceutical compositions. Concentration may vary over a broad range, typically 0.01-10 wt-%.
In a further embodiment, the invention also provides compositions as described herein that comprise an oxidizing agent (5) as described herein and HA, preferably each in an effective amount. It is surprisingly found that a concurrent application of an oxidizing agent and HA has excellent effect in preventing and reducing scarring, stipulating from cuts and infection diseases and disorder. Scars, also called cicatrices, are areas of fibrous tissue (fibrosis) that replace normal skin (or other tissue) after injury that stems from cuts, infections such as acne, boils, eczema, cellulitis, pustule, chromobateriosis, etc., diseases such as dermatosis, necrosis, etc., amongst others. A scar results from the biologic process of wound repair in the skin. Scar tissue is not identical to the tissue that it replaces and is usually of inferior functional quality. Skin scars are less resistant to ultraviolet radiations. Sweat glands and hair follicles do not grow back within scar tissue. The toxins that the infection causing bacteria and fungi secret, stimulate our body further for rapid healing through generating collagen enriched fibrous scar tissue.

**Isotonic agents:** One or more isotonic agents may be employed in the inventive composition. Isotonic agents are known in the field; they are often useful to be incorporated in a oral care compositions and pharmaceutical compositions in amounts which impart to the infusion solution the same or essentially the same osmotic pressure as body fluid. Suitalbe isotonic agents include sodium chloride, mannitol, dextrose, glucose. These agents reside in the aqueous phase of the composition. For oral care applications, most preferred one is sodium chloride. Concentration may vary over a broad range, typically 0.05-1 wt-%.

**Lytic Bacterio-phages:** One or more lytic Bacterio-phages may be employed in the inventive composition. Lytic Bacterio-phages are known in the field; they are suitable for treating and preventing bacterial infections such as oral and skin infections. Bacterio-phages may be selected from the group consisting of *Streptococcus mutans,* such as Phage M102, Phage E10, Phage F1.

**Mineralizing / re-mineralizing agents:** One or more (re)-mineralization agents may be employed in the inventive composition. (Re)-mineralization agents for dental applications are known in the field. (Re)-mineralization agents prevent formation of carious lesion, lowers tooth hypersensitivity by acting as precursors of apatite materials when applied in the oral cavity. (Re)-mineralization agents include calcium ion releasing agent, phosphate ion releasing agent and fluoride ion releasing agent. Suitable calcium ion releasing agents are the water soluble salts of calcium. Suitable phosphate ion releasing agents are the water soluble salts of phosphates. Suitable fluoride ion releasing agents are the water soluble alkali metal and ammonium fluorides, fluorophosphates, fluorozirconates, etc. Suitable (re)-mineralization agents are disclosed in WO2010/054494, particularly on pages 18-19 and 22-24. They are present in the aqueous phase.

**Nutritients:** One or more nutritients may be employed in the inventive composition. Suitable nutrients are known in the field and include water soluble vitamins and lipophilic vitamins. Suitable water soluble vitamins are selected from the group consisting of vitamin C (Ascorbic acid), B1 (Thiamine), B2 (Riboflavin), B3 (Niacin), B5 (Pantothenic acid), B6 (Pyridoxine), B7 (Biotin), B9 (Folic acid), B12 (Cyanocobalmine), and their water soluble precursors. Suitable fat soluble (lipophilic) vitamins are selected from the group consisting of vitamin A (Retinol), D (Cholecalciferol), E (Tocopherol), K (phylloquinone) and their lipophilic precursors. This lipophilic class of vitamins constitute important lipophilic ingredients (4), which are solubilised in the core of the inventive micelles.
Concentration may vary over a broad range, typically 0.01-15 wt-%.

**Opacifier:** One or more opacifiers may be employed in the inventive composition. Opacifiers are known in the field; they reduce or eliminate the clear or transparent appearance of a composition or end product. An opacifier often is included in a composition to mask an undesirable esthetic property, such as to improve the color of a composition that is darkened due to the presence of a particular ingredient, or to mask the presence of particulate matter in the composition., Opacifiers also are included in aqueous compositions to improve the esthetics and consumer acceptance of an otherwise esthetically-unacceptable composition. Further, an opacifier may impart other advantageous properties to a composition, such as thickening, suspending and emulsifying properties. Opacifiers commonly are included in hair care compositions, skin care compositions and topical pharmaceutical compositions. It is known that suspendable agents and gelling agents/thickeners may also impart opacity to micellar compositions.
Suitable opacifiers may be selected from a number of different chemical classes including inorganic compounds, (e.g. aluminum and magnesium salts) and organic compounds, (e.g. fatty alcohols, fatty amines, fatty amine oxides, fatty esters and polymeric compounds). A representative listing of opacifiers is found in the CTFA Cosmetic Ingredient Handbook, J. Nikitakis, ed., The Cosmetic, Toiletry and Fragrance Association, Inc., Washington, D.C., 1988, at page 75.

**Preservatives:** One or more preservatives may be employed in the inventive composition. Preservatives are known in the field to preserve a composition from bacterial, fungal and other microbial contamination over the shelf-life. A group of preservatives is the oxidizing agent as defined herein. Another group of preservatives are naturally occurring essential oils and extracts with. Suitable preservatives may be selected from the group consisting of Xylitol, Thymol, Eucalyptol, Euganol, Oil of Colve, Sweet Birch Oil, Wintergreen Oil, D-Limonene, Mentha Paprita L oil, Spearmint oil. Concentration may vary over a broad range, typically 0.01-8 wt-%.
Another group of preservatives are conventional preservatives. Suitable preservatives may be selected from the group consisting of Methyl Paraben, Ethyl Paraben, Propyl Paraben, Domiphene Bromide, Salicylanide, Zinc ions, Stannous ions, Octenidine, Delmopione, Octapinol, Chlorhexidin, Hexetidin, Cetylpyridinium chloride, Cetylpyridinium bromide, biguanides. A particularly preferred preservative is Polyaminopropyl Biguanide, since it is considered a gentle and effective anti-microbial substance that acts against gram negative bacteria, gram positive bacteria, yeast and fungi over a wide range of pH. Concentration may vary over a broad range, typically 0.1-5 wt-%.

**Suspendable agents:** One or more suspendable agents may be employed in the inventive composition. Suitable suspendable agents may be chosen from anhydrous or hydrous humectants. Solid materials which can be suspended in a colloid system("powders") are considered suspendable materials. Suspendable materials find applications as abrasive, polishing agent and coloring/pigmenting agent. Generally, any abrasive known in the field may be applied. Suitable abrasives are selected from the group consisting of silica (such as fumed silica, anhydrous silica, hydrated silica), Brushite CaHPO₄·2H₂O, Dicalcium phosphate, Kaolin, Aragonite, Calcite, white Pearl powder, Baking Soda, Calcium Carbonate, Acrylic glass (such as PM/MA) and Aluminium hydroxide. The amount of abrasive used may vary in a broad range and can be determined by simple routine experiments, suitable are, for example 1-25% of abrasive in the composition. Abrasives are particular preferred when the oral composition is formulated as a toothpaste or gel. Oral care application, abrasives are preferred to be associated with high pellicle cleaning ratio (PCR) and low radioactive dentine and enamel abrasions (RDA and REA) in order to produce higher cleaning efficacy at lower erosive damages to the teeth. Some specific examples advantageous for oral care suitable includes, but not limited to, SYLODENT and SYLOBLAC series (supplied by GRACE), Zeodent 103, Zeodent 113 and Zeodent 124 (supplied by HUBER), Sident series (supplied by Degussa), etc. Polishing agents can be low-structured silica, metal oxide powders such as Titanium dioxide, Zinc oxide, Aluminium oxide with very low particle size ranging preferably in nanometers. Use level may vary over a wide range, typically 0.1-10% and depending on the field of application.
Pigment materials may be suspended in the inventive compositions, e.g. in gel and emulsion compositions, to give coloration. Suitable pigment / coloring agent may be selected from the group consisting of TiO2, ZnO, Al2O3, alkaline earth and periodic table group 12 metal carbonates, alkaline earth and periodic table group 12 metal sulphates. Preferably, the above pigment materials have particle-size between micro- and nano- meters. Some specific examples include, but not limited to, Candurin Silver Fine, Candurin Silver Sheen, Candurin Blue Shimmer, Candurin Green Shimmer, Timiron, Colorona, Ronastar and Xirona, supplied by Merck Cosmetics Switzerland.

**Sweeteners:** One or more natural or artificial sweeteners may be added to the inventive composition. The addition of sweeteners is commonly done to improve consumer acceptance; any sweetener or combination of sweeteners known in the field may be used. Preferred are sweeteners that are stable towards oxidizing conditions and soluble in aqueous media, such as Acesulfame-K. Sweeteners of edulcorant type are most preferred. Non-limiting examples of preferred sweeteners include alkali Acesulfame, alkali Saccaharin, Aspartame, Levulose, Thaumatine, D-Tryptophan, Dihydrochalcones, Cyclamate, Xylitol, Dextrose, etc.
Concentration may vary over a broad range, typically 0.05-10 wt-%.

**Thickener / Binder:** One or more thickeners / binder may be employed in the inventive composition. Suitable binders may be chosen from an inorganic class and / or organic class of binders, or a mixture thereof. Concentration may vary over a broad range, typically 0.001 to 50%. In this context, a thickener / binder is indentified as a viscosity enhancing agent. Typically, when applied in an aqueous or non-aqueous medium, it results in the formation of gels that exhibit thixotropic properties.
The inorganic class comprises thickening silicas (hydrated, precipitated, fumed, etc.), colloidal silicas, alkali-earth metal aluminium silicates, natural and synthetic clays. Zeodent 165, Sident 22S, Syloblanc 34 and the like are proven useful thickening silica, with use level 0.1 to 40%. Non-limiting examples of the at least one viscosity-enhancing agent include magnesium aluminum silicates, smectite clays, and an amorphous clay mineral, such as allophone; two-layer type crystalline clay minerals, such as equidimensional crystal, kaolinite, and nacarite; elongate crystals, such as halloysites; three-layer type crystalline clay minerals, such as sodium montmorillonite, calcium montmorillonite, sauconite, vermiculite, non-tronite, saponite, hectorite, and bentonite (Optigel); chain structure crystalline clay minerals, such as attapulgite, sepiolite, and palygorskite; sodium lithium magnesium silicate (Laponite) and mixtures thereof. For aqueous formulations, Laponite and Optigel, supplied by Rockwood additives, have proven promising, although they are not preferably used in the compositions wherein concentration of bi-valent metal ions is higher than 6%. For w/o type compositions, Thixogel type of clay-gelling agents, supplied by Rockwood has proven very promising. Thixogel based compositions have shown to work with high electrolyte (30%) as well as high concentration of calcium/bi-valent metal ions (15%). The use level of said clay gelling agents may vary between 1 and 15% depending on the compositions. For high electrolyte, as well as high calcium/bivalent metal ion content compositions, the leading viscosity enhancer agent is one or a mixture of thickening silicas, either with a combination of clay gelling agents and / or organic class of thickener.
The organic class falls into two major families; non-ionic and ionic. Non-ionic class comprises (C₂₋₆)alkylene oxide modified (C₁₋₆)alkylcellulose ethers, for instance, hydroxyethyl cellulose HEC (Natrosol and modified Natrosol type of gelling agents supplied by CP Kelco) hydroxypropyl cellulose, hydroxypropyl methylcellulose; Xanthan gum (Keldent, Keltrol from CP Kelco); Guar gum (calcium ions and / or electrolytes help gelling/thickening); gum Karaya; gum Tragacanth; Irish moss; gum Arabic; cornstearch; polyvinylpyrrolidone; Copolymers like Methyl vinyl ether/maleic anhydride copolymer (PVM/MA); polyethylene glycol (PEG) block polymers; etc. The use level may vary between 0.1 and 15% depending on the compositions. The ionic class comprises anionic and cationic types of organic thickening agents. Anionic type includes carbopols, especially the ones that are designed to be stable and compatible with electrolytes, for example Carbopol Ultrez 21, Carbopol ETD 2020 and Carbopol Aqua SF-1; Walen Gum (compatible with calcium); gellan gum; alkali metal carrageenan; Alkali and alkali earth metal carboxymethyl cellulose (CMC) etc. Cationic type includes guar hydroxypropyl trimonium chloride, and the like. The use level of the ionic binder may vary over a wide range between 0.1 and 20 wt-%.
It has been found that a combination of said inorganic and organic thickening agents, in 300:1 to 2:1 %-weight ratio, is advantageous in formulating suitable end-products, for both personal care and professional care (higher electrolyte and higher-valent metal ion contents), with reasonable stability, shelf life, desired functionalities, viscosity, density, rheological properties, surface texture, feeling, etc., which are important characteristics of an end-user product, ready to sell.
Surfactant thickeners are another class of thickener / binder suitable for the inventive compositions. The preferred ones are non-ionic and zwitterionic surfactants. The non-ionic ones are already included in the list of non-ionic surfactants for formulation. In general, they are solid or semi-solid. They, in general but not limited to, require heating above their melting points in order to be incorporated in a composition. They are particularly capable of thickening/gelling a wide range of liquid organic mediums (such as esters, hydrocarbons, alcohols (mono- as well as poly-ols; e.g. ethanol, such as glycols, polyglycols, glycerol, etc.), carbon based oils (such as triglycerides), silicon oils, etc., as well as stabilizing and binding aqueous and oil phases.
Concentration may vary over a wide range, typically 0.1-40 wt-%.
Ethyl cellulose, propyl cellulose, alkali earth metal acetates, alkali and alkali earth metal salts of fatty acids, polawax, emulsifying wax are further non-limiting examples of thickener/binders suitable for the inventive compositions.

In an advantageous embodiment, the invention relates to a composition as described herein which is free of parabenes. Such compositions are particular compatible with sensitive users.

In an advantageous embodiment, the invention relates to a composition as described herein which is free of monohydric-P105387EP01 desc 2016-09.docx alcohols, particularly ethanol. Such compositions are particular compatible with sensitive users.

In an advantageous embodiment, the invention relates to a composition as described herein which is free of aliphatic ethers, particularly dialkyl ethers with 12-24 C-atoms. Such compositions are particular useful for oral care products and / or for compositions comprising oxidizing agents.
In a **second aspect,** the invention relates to end products adapted to the professional and / or private user in cosmetic applications, household applications and / or pharmaceutical applications. As indicated above, the inventive compositions may be directly used by the end-user or suitably formulated to obtain an end-product. Suitable formulations of the inventive composition are any liquid, semi-solid or solid formulations known in the field. Liquid end products include sprays and rinses. Semi-solid end products include pastes, gels, creams and lotions. Solid end products include powders, chewing gums, lozzanes, candies, tablets, and dragees. As already outlined, the inventive compositions and end products may be used in a large variety of applications, including oral care, skin care, hair care, household and pharmaceutical applications; corresponding end products may be adapted according to these applications in analogy to known products in that field, particularly by adapting suitable amounts of lipophilic ingredients, hydrophilic ingredients and further excipients. The invention consequently also provides a composition as described herein which is (1) an oral care composition (such as a mouth spray, cream, gel, paste); (2) a skin care composition, (3) a hair care compositions; (4) a pharmaceutical composition; or (5) a household cleaning composition.
Further, the end-products may be adapted for use in professional or consumer applications.

Depending on field of application, each composition furnishes a group of additives, including the lipophilic ingredients (4), which are required to be incorporated into the inventive composition, which then in combination with additives (5) - (8) forms the end product.
Further, any end product may be a single system or a dual or a multiple systems of delivery vehicle.
Furthermore, any end product may be applied in mono- or dual- or multiple doses, including simultaneous or sequential mode of application.

**Oral Care Compositions:** In an advantageous embodiment, the invention thus relates to a composition as described herein, which is an oral care composition, including toothpaste / toothgel / dental creams (Semi-solid), mouthwash / mouthspray / mouthrinse / oral solution / oral Lotion / oral drop (Liquid), dental implements / bleaching kit (Semi-solid), chewing gum / candy / tooth powder (Solid).

Additives for oral care compositions include, although are not limited to, abrasives, thickeners / gelling agents / viscosity enhancers, humectants, pH buffering agents / pH regulators, anti-plaque agents, an anti-carious agents, anti-tartar / anti-calculus agents, polishing agents, foaming agents / mild detergents, bleaching agents, tooth mineralizing agents, sweeteners, oral isotonic agents, nutrient agents, flavoring / fragrance agents, coloring agents, enzymes. Within these additives, the lipophilic ingredients (4) include flavoring agent, coloring agent, nutrient agent (such as fat soluble vitamins), enzymes (such as lipophilic enzymes); the excipients (8) include abrasive and polishing agents; whereas hydrophilic ingredients (7) include the rest of the additives.

In one advantageous embodiment, the inventive composition is formulated as a mouth spray, mouth wash, mouth rinse, gurgling rinse. In one particularly advantageous embodiment, the inventive composition is formulated as a mouth spray. Such formulations are suitable for cosmetic and therapeutic treatment, such as described below. In a further advantageous embodiment, the inventive composition is formulated as a gurgling rinse (or mouthwash). Such formulations are particularly suitable for the treatment of flu and bacterial infections to throat and larynx.

In an advantageous embodiment, the invention relates to an oral care composition as described herein, having a viscosity similar to water; typically in the range between 2.5 10⁻⁴ and 1.5 10⁻³ Pas.

**Skin care compositions:** In an advantageous embodiment, the invention thus relates to a composition as described herein, which is a skin care composition, including creams, lotions.

**Hair care compositions:** In an advantageous embodiment, the invention thus relates to a composition as described herein, which is an hair care composition, including shampoos.

**Pharmaceutical compositions:** In an advantageous embodiment, the invention thus relates to a composition as described herein, which is a pharmaceutical composition.

**Household cleaning compositions:** In an advantageous embodiment, the invention thus relates to a composition as described herein, which is a cleaning composition, particularly for solid surfaces.

In a **third aspect**, the invention relates to the use of a compound or composition as described herein in cosmetic applications, household applications and / or therapeutic applications.

In one embodiment, the invention provides a composition as disclosed herein as pharmaceutical, particularly for the treatment, prevention and / or delay of progression of halitosis. The term "halitosis" is known in the field; it also includes oral malodor and bad breath. The compositions and methods of treatment of the present invention are particularly effective for treating or preventing halitosis. Halitosis may be caused from consumption of disadvantageous foods or drinks; from smoking. Halitosis may also be caused from high plaque count. Treatment may be effective irrespective of the underlying cause.

In one further embodiment, the invention provides a composition as disclosed herein as pharmaceutical, particularly for the treatment, prevention and / or delay of progression of diseases or conditions of the oral cavity. The term "diseases or conditions of the oral cavity" is known in the field; it particularly denotes periodontal disease, gingivitis, periodontitis, periodontosis, adult and juvenile periodontitis, and other inflammatory conditions of the tissues within the oral cavity, plus caries, necrotizing ulcerative gingivitis, and other conditions such as herpetic lesions, and infections that may develop following dental procedures such as osseous surgery, tooth extraction, periodontal flap surgery, dental implantation, and scaling and root planing. Also specifically included are dentoalveolar infections, dental abscesses (e.g., cellulitis of the jaw; osteomyelitis of the jaw), acute necrotizing ulcerative gingivitis (i.e., Vincent's infection), infectious stomatitis (i.e., acute inflammation of the buccal mucosa), and Noma (i.e., gangrenous stomatitis or cancrum oris). Oral and dental infections are more fully disclosed in Finegold, Anaerobic Bacteria in Human Diseases, chapter 4, pp 78-104, and chapter 6, pp 115-154 (Academic Press, Inc., NY, 1977). The compositions and methods of treatment of the present invention are particularly effective for treating or preventing periodontal disease (gingivitis and/or periodontitis) and resulting breath malodor. Further, the term "diseases or conditions of the oral cavity" covers systemic and local causes; it thus includes underlying diseases, disorders or malfunctions, such as periodontal diseases and disorders; tongue and throat diseases associated infections, viral infections (such as influenza), related bad-breath, shore throat problems, and bacterial infections to throat and larynx. Further, the term "disease or condition of the oral cavity" covers tooth whitening, tooth discoloration, tooth staining, tooth decay and tooth hypersensitivity.

In one further embodiment, the invention provides a composition as disclosed herein as pharmaceutical, particularly for the treatment, prevention and / or delay of progression of diseases or conditions of the skin. The term "diseases of the skin" is known in the field; it particularly denotes acne.

In one further embodiment, the invention provides the use of composition as disclosed herein for the treatment, prevention and / or delay of progression of halitosis and / or diseases or conditions of the oral cavity and/or acne.

In one further embodiment, the invention provides the use of composition as disclosed herein for the manufacturing of a pharmaceutical for the treatment, prevention and or delay of progression of halitosis and / or diseases or conditions of the oral cavity and / or diseases or conditions of the skin.

In one further embodiment, the invention provides a method of treating halitosis and / or diseases or conditions of the oral cavity and / or diseases or conditions of the skin in a subject in need thereof comprising the step of contacting the oral cavity or the skin of said subject with an effective amount of a composition as described herein.

In one further embodiment, the invention provides the use of composition as disclosed herein for the cosmetic treatment of oral malodor and/or for cosmetic treatment of the skin ("skin care") and/or for cosmetic treatment of the hair ("hair care").

In one further embodiment, the invention provides a method of cleaning the oral cavity or the skin or the hair of a subject in need thereof comprising the step of contacting the oral cavity or the skin or the hair with an effective amount of a composition as described herein.

In one further embodiment, the invention provides the use of composition as disclosed herein for cleaning solid surfaces ("household applications"), such as sanitary facilities, floors, tableware, cooking devices, cutlery.

In one further embodiment, the invention provides a method of cleaning solid surfaces comprising the step of contacting the surface with an effective amount of a composition as described herein.

The cosmetic/cleaning effects and therapeutic effects of the composition as described herein are clearly different. While a cosmetic/cleaning effect (such as treatment of halitosis, cleaning of surfaces) is directly achieved by one single application, a therapeutic effect (such as treatment of diseases of the oral cavity) is obtained by an application over a prolonged period, e.g. daily application over one week. In a further advantageous embodiment, the cosmetic treatment comprises the step of applying once one or two doses of 0.1-1 ml, e.g. 0.25 ml, of the inventive composition. In a further advantageous embodiment, the therapeutic treatment comprises the step of multiple applications of one or two doses of 0.1 - 1 ml, e.g. 0.25 ml, of a composition as described above. Such multiple applications include applications once a day, twice a day, or 3 times a day over a period of 2 or more days, such as 2 days, 5 days, 1 week, 2 weeks, 1 month.

In a **fourth aspect,** the invention relates to the manufacture of compositions as described herein. The manufacturing of micellar compositions is known in the field and generally comprises the combination of the required constituents at room temperature or elevated temperatures using standard equipment. Typically, the oil phase is added to the water phase. As outlined below, the process of manufacturing inventive micelles also includes as novel sequence of individual steps. The invention therefore not only provides new and inventive micellar compositions, but also a new and inventive manufacturing process therefore and micellar compositions obtained by such process.

In one embodiment, the invention relates to a method for manufacturing a composition as described herein, comprisinq the steps of (a) providing a non-aqueous phase by combining constituents (1), (2), optionally (4) and (3), in this order; (b) providing an aqueous phase by combining constituents (5), (6), (7) and optionally (8) with water; (c) combining said non - aqueous phase with said aqueous phase to obtain a micellar composition and (d) optionally combining the obtained micellar composition with further components to obtain an end product.
In a preferred embodiment, step (a) is performed as follows: Ionic (1) and non-ionic (2) surfactants are blended by thorough agitation (optionally with heating) for at least 15-30 mins, followed by addition of lipophilic ingredients with agitation (optionally with heating) for at least 30 mins and followed by addition of co-surfactant (3) with agitation for 30 mins. It is believed that the micelles of the inventive composition are actually formed in these 3 steps. Without these 3 consecutive manufacturing steps, one cannot ensure the inventive mixed-micellar composition is formed. Blending surfactants (especially for a group of non-ionic surfactants) is known in the field. However the micelles of the prior art distinguish from the present inventive micelles, because the literature does not show any record for blending thoroughly ionic and non-ionic surfactants, followed by loading with lipophilic ingredients and finally followed by accommodation of co-surfactants at the interface to complete formation of inventive micelles with thick and relaxed surface/interface. It is also noted that addition of an ionic surfactant to a composition comprising a non-ionic surfactant and a group of lipophilic ingredients at any manufacturing step (as disclosed in the prior art discussed above) does not produce micelles according to the present invention; not even a few are formed.
Depending on the constituents and the equipment used, the manufacturing takes place at 15 - 85 °C, preferably at room temperature. Combining materials may be accomplished according to standard procedures, e.g. by slowly dosing one material into the other while agitating.

A more specific manufacturing process for producing the inventive micellar compositions comprises the following steps:
(a1) First, charge mixer container with ionic (1) and non-ionic (2) surfactants. (a2) Blend the mixture thoroughly with agitation and apply heat if required for between 30 minutes and 2 hours. This produces "parent" mixed micelles. (a3) Add lipophilic ingredients (4) to mixture and continue agitating, preferably at least for 30 minutes, until a uniform, transparent or translucent mixture is obtained. This produces "loaded mixed micelles". (a4) Add co-surfactant (3) and mix thoroughly with agitation until a clear homogeneous mixture is obtained, preferably for at least 30 minutes. The thus obtained non-aqueous phase is referred to as "micellar phase" or "phase A" herein. Steps (b), (c) and (d) may be performed as outlined above.

A more specific manufacturing process for producing the inventive micellar compositions comprises the following steps:
(c) Combining the aqueous and non-aqueous phase is accomplished by introducing slowly, under agitation, the aqueous phase of step (b) to the non-aqueous phase of step (a). Steps (a), (b) and (d) may be performed as outlined above.

General description for manufacturing (micro)-emulsion type compositions: (1) Separate preparation of an aqueous phase, referred to as "aqueous phase" or "phase B" herein, by dissolving all water soluble ingredients in water in an appropriate sequence with application of agitation and heating if necessary, except the oxidizing agent. pH is adjusted in phase A as to produce the desired pH of the end-product (2) Addition of phase A into phase B with agitation. (3) Finally addition of the oxidizing agent to the mixture with agitation.

General description for manufacturing semi-solid type compositions: (1) Prepare a homogeneous gel by dispersing a gelling agent into the dispersion medium, which is either water or hydro-alcohol (mixture of water and poly-ols) or poly-ol or organic solvent (w/o case), preferably a mixture of water and poly-ol in case of o/w composition, with agitation and heating/cooling if necessary. (2) Transfer the gels, if there are more than one, to the mixer, add the co-surfactant / constituent (2) if it was not added in step 1, and mix thoroughly under vacuum with agitation until smooth and homogeneous. (3) Prepare separately an aqueous phase by dissolving all water soluble ingredients, except any oxidizing agent, in an appropriate sequence in water, with application of agitation and heating, if necessary, such that it will produce the desired pH of the end-product. (4) Add the aqueous phase to the mixer and mix under vacuum with agitation for 30 minutes to one hour. (5) Add suspendable agent (constituent (8)), if any, to the mixer and mix first slowly, preferably between 100 and 400 rpm of propeller speed, as to incorporate the particles of suspendable agent into the composition for 15 minutes to 30 minutes, followed by rapidly with propeller speed, preferably between 600 and 1200 rpm while between 600 and 900 rpm for toothpaste/toothgel/dental cream, under vacuum for 30 minutes to 5 hours, with monitoring the temperature at desired level, preferably within 20 and 30C, (5) Make up the temperature of the resulting mixture to 20-25C. (6) Prepare separately the phase A following the steps a, b and c, if the co-surfactant is used up, otherwise steps a, b, c and d, as disclosed. (7) Add the phase A to the mixer and mix first slowly with propeller speed between 100 and 400 rpm for 10 to 30 minutes and then rapidly with propeller speed between 500 and 900 rpm under vacuum, (8) Add the foaming agent (constituent (7)), if any, preferably in dilute form; an aqueous solution form if possible, and mix under vacuum with moderate propeller speed ranging between 400 and 900 rpm preferably between 400 and 600 rpm for toothpaste/toothgel/dental cream. Finally, (9) add the oxidizing agent, preferably in dilute form such as an aqueous solution thereof, and mix under vacuum with moderate propeller speed ranging between 400 and 900 rpm preferably between 400 and 600 rpm for toothpaste/toothgel/dental cream.

In the manufacturing, it should be avoided to adjust pH of the composition after the incorporation of "phase A", as it will lead to variations in ionic strength of the composition which is associated with disadvantageous micellar reorganization, micellar fragmentation, micellar migration, and hence confrontation between lipophilic ingredients and aqueous phase.

In the manufacturing, it should be avoided to add electrolytes, such as oxidizing agent and foaming agent, in dilute form after the incorporation of "phase A" in the composition.

In the manufacturing, it should be avoided to incorporate "phase A" below 30°C, as to avoid probabilities of micellar collision, micellar migration and micellar fragmentation at higher temperature.

The following **examples** further illustrate the invention without any limitation.

### Manufacturing Process:

The compositions according to ex. 1 - 9 are manufactured as outlined above using the ingredients as specified. The ingredients are commercially available and used as obtained.

**Ex-1: (micro)-emulsion**

| **Ingredients** | **Conc. %** | |
|---|---|---|
| Pluronic L 35 | 4 | |
| Pluronic 10R5 | 1 | |
| Laureth-11 Carboxylic acid | 2 | |
| Glycerin | 15 | A |
| Propylene Glycol | 10 | |
| Eucalyptol | 0.1 | |
| Wintergreen oil | 0.1 | |
| Flavor | 1 | |
| Deionized Water | 57.589 | |
| KOH | 2.95 | |
| Boric Acid | 2.73 | |
| K2HPO4 | 1.04 | |
| KH2PO4 | 0.016 | |
| Sodium Chlorite 80% | 0.625 | B |
| Acesulfame-K | 0.9 | |
| KNO3 | 0.25 | |
| KCl | 0.1 | |
| NaCl | 0.1 | |
| Sodium Monofluorophosphate | 0.5 | |
| | 100 | |

The obtained composition is clear and colorless, free of ethanol and suitable as mouthspray; pH = 9.15.

**Ex-2: (micro)-emulsion**

| **Ingredients** | **Conc. %** | |
|---|---|---|
| PPG-26-Buteth | 3 | |
| Laureth-11 Carboxylic acid | 3 | |
| Glycerin | 15 | A |
| Propylene Glycol | 10 | |
| Eucalyptol | 0.1 | |
| Wintergreen oil | 0.1 | |
| Flavor | 1 | |
| Deionized Water | 58.469 | |
| KOH | 3.07 | |
| Boric Acid | 2.73 | |
| K2HPO4 | 1.04 | |
| KH2PO4 | 0.016 | |
| Sodium Chlorite 80% | 0.625 | B |
| Acesulfame-K | 0.9 | |
| KNO3 | 0.25 | |
| KCl | 0.1 | |
| NaCl | 0.1 | |
| Sodium Monofluorophosphate | 0.5 | |
| | 100 | |

The obtained composition is clear and colourless, free of ethanol and suitable as mouthspray; pH = 9.2/9.19.

**Ex-3: (micro)-emulsion**

| **Ingredients** | **Concentration %** | |
|---|---|---|
| Polysorbate 20 | 5 | |
| Cocamine Oxide (30%) | 1 | |
| Glycerin | 15 | |
| Propylene Glycol | 10 | A |
| Eucalyptol | 0.1 | |
| Wintergreen oil | 0.1 | |
| Flavor | 1 | |
| Deionized Water | 58.669 | |
| KOH | 2.87 | |
| Boric Acid | 2.73 | |
| K2HPO4 | 1.04 | |
| KH2PO4 | 0.016 | |
| Sodium Chlorite 80% | 0.625 | B |
| Acesulfame-K | 0.9 | |
| KNO3 | 0.25 | |
| KCl | 0.1 | |
| NaCl | 0.1 | |
| Sodium Monofluorophosphate | 0.5 | |
| | 100 | |

The obtained composition is clear and colorless, free of ethanol and suitable as mouthspray; pH = 9.12.

**Ex-4: (micro)-emulsion**

| **Ingredients** | **Conc. %** | |
|---|---|---|
| Polysorbate 20 | 5 | |
| Laureth-11 Carboxylic acid (86%) | 3 | |
| Glycerin | 15 | A |
| Propylene Glycol | 10 | |
| Eucalyptol | 0.1 | |
| Wintergreen oil | 0.1 | |
| Flavor | 1 | |
| Deionized Water | 56.789 | |
| KOH | 2.75 | |
| Boric Acid | 2.73 | |
| K2HPO4 | 1.04 | |
| KH2PO4 | 0.016 | |
| Sodium Chlorite 80% | 0.625 | B |
| Acesulfame-K | 0.9 | |
| KNO3 | 0.25 | |
| KCl | 0.1 | |
| NaCl | 0.1 | |
| Sodium Monofluorophosphate | 0.5 | |
| | 100 | |

The obtained composition is clear and colorless, free of ethanol and suitable as mouthspray.

**Ex-5: (micro)-emulsion**

| **Ingredients** | **Conc.%** | |
|---|---|---|
| Pluronic L 35 | 4 | |
| Pluronic 10R5 | 1 | |
| Lauryl Dimethy Amine Oxide (30%) | 2 | A |
| Glycerin | 15 | |
| Propylene Glycol | 10 | |
| Eucalyptol | 0.1 | |
| Wintergreen oil | 0.1 | |
| Flavor | 1 | |
| Deionized Water | 57.589 | |
| KOH | 2.95 | B |
| Boric Acid | 2.73 | |
| K2HPO4 | 1.04 | |
| KH2PO4 | 0.016 | |
| Sodium Chlorite 80% | 0.625 | |
| Acesulfame-K | 0.9 | |
| KNO3 | 0.25 | |
| KCl | 0.1 | |
| NaCl | 0.1 | |
| Sodium Monofluorophosphate | 0.5 | |
| | 100 | |

The obtained composition is clear and colorless, free of ethanol and suitable as mouth spray.

**Ex-6: (micro)-emulsion**

| **Ingredients** | **Conc %** | **comment** |
|---|---|---|
| Pluronic L 35 | 3.75 | |
| Pluronic 10R5 | 0.75 | |
| Laureth-11 Carboxylic acid (86%) | 1 | |
| KOH | 0.3 | A |
| Glycerin | 24 | |
| Eucalyptol | 0.05 | |
| Wintergreen oil | 0.05 | |
| Cool Spearmint C123 | 0.25 | |
| Deionized Water | 65.947 | |
| KOH | 0.35 | |
| Boric Acid | 0.8 | |
| K2HPO4 | 0.52 | |
| KH2PO4 | 0.008 | |
| Sodium Chlorite 80% | 0.625 | B |
| Acesulfame-K | 0.8 | |
| KNO3 | 0.25 | |
| KCl | 0.1 | |
| NaCl | 0.1 | |
| Sodium Monofluorophosphate | 0.35 | |
| | 100 | |

The obtained composition is clear and colorless, free of ethanol and suitable as mouthwash.

**Ex-7: Semi-solid**

| **Ingredients** | **Conc %** | **comment** |
|---|---|---|
| Water | 18.714 | |
| Glycerin (99.5%) | 12.5 | |
| Carbopol ETD 2020 | 0.8 | |
| KOH | 0.3 | |
| Water | 2.5 | |
| Keldent | 1.5 | Gel |
| PEG 400 | 6 | |
| Glycerin (99.5%) | 12.5 | |
| Water | 7.5 | |
| Water | 7.5 | |
| Tetrapotassium Pyrophosphate | 0.8 | |
| KOH | 1 | B |
| Boric Acid | 1.356 | |
| K2HPO4 | 1.04 | |
| KH2PO4 | 0.016 | |
| Acesulfame-K | 0.9 | |
| KNO3 | 0.8 | |
| KCl | 0.1 | |
| NaCl | 0.1 | |
| Sodium Monofluorophosphate | 0.5 | |
| Syloid AL-1FP | 2.45 | gel |
| Syloblanc 34 | 10 | |
| Candurin Blue | 0.25 | |
| Candurin Green | 0.25 | |
| Mica | 0.3 | |
| Pluronic L35 | 2 | |
| Pluronic 10R5 | 1 | |
| Laureth-11 Carboxylic acid (86%) | 2 | |
| KOH | 0.1 | A' |
| Water | 0.3 | |
| Flavor | 1 | |
| Eucalyptol | 0.1 | |
| Wintergreen oil | 0.1 | |
| Water | 1.374 | |
| Sodium Lauryl Sarcosinate | 0.35 | mild detergent oxid. agent |
| Sodium Chlorite 25% | 2 | |
| | 100 | |

In this example, a gel is formed, by combining the above identified ingredients in the given order. Phase B is manufactured by combining the above identified ingredients in the given order to obtain a homogeneous phase. Both phases are combined and the remaining components are added to obtain gel, which may be considered a thickened co-surfactant. Phase A' (which is phase A without co-surfactant) is manufactured as described above. Phase A', gel' are combined and the remaining ingredients are added to obtain an opal product suitable as toothpaste; pH = 8.0.

**Ex-8: Semi-solid**

| **Ingredients** | **Conc %** | **comment** |
|---|---|---|
| Water | 18.714 | |
| Propylene Glycol | 12.5 | |
| Carbopol ETD 2020 | 0.8 | |
| KOH | 0.3 | |
| Water | 2.5 | |
| Keldent | 1.5 | |
| PEG 400 | 6 | |
| Glycerin (99.5%) | 12.5 | |
| Water | 7.5 | |
| Water | 7.5 | |
| Tetrapotassium Pyrophosphate | 0.8 | |
| KOH | 1 | |
| Boric Acid | 1.356 | |
| K2HPO4 | 1.04 | |
| KH2PO4 | 0.016 | |
| Acesulfame-K | 0.9 | |
| KNO3 | 0.8 | |
| KCl | 0.1 | |
| NaCl | 0.1 | |
| Sodium Monofluorophosphate | 0.5 | |
| Syloid AL-1FP | 2.45 | |
| Syloblanc 34 | 10 | |
| Candurin Blue | 0.25 | |
| Candurin Green | 0.25 | |
| Mica | 0.3 | |
| Pluronic L35 | 2 | |
| Pluronic 10R5 | 1 | |
| Laureth-11 Carboxylic acid (86%) | 2 | |
| KOH | 0.1 | |
| Water | 1.3 | |
| Flavor | 1 | |
| Eucalyptol | 0.1 | |
| Wintergreen oil | 0.1 | |
| Water | 1.374 | |
| Sodium Lauryl Sarcosinate | 0.35 | |
| Sodium Chlorite 25% | 1 | |
| | 100 | |

In this example, the procedure of ex. 7 is followed to obtain an opal product, suitable as toothpaste; pH = 8.0.

**Ex-9: Semi solid**

| **Ingredients** | **Conc %** | **comment** |
|---|---|---|
| Water | 15.15 | |
| Natrosol 250 MR | 0.8 | (gelling·agent) |
| Glycerin (99.5%) | 15 | |
| PEG 400 | 4 | |
| Keldent | 1.5 | gel |
| Sorbitol (70%) | 15.75 | |
| Water | 11.55 | |
| Syloid AL-1FP | 2.45 | |
| Syloblanc 34 | 10.6 | |
| Aluminum Hydroxide | 0.2 | |
| Candurin Blue Shimmer | 0.1 | |
| Mica | 0.6 | |
| Brushite | 2.25 | suspendable |
| Xylitol | 0.5 | |
| ZnO | 0.1 | |
| Zinc Citrate | 0.5 | |
| Water | 7 | |
| KNO3 | 1 | |
| Calcium Glycerophosphate | 2 | |
| Papain | 0.5 | B |
| Polyaminopropyl biguanide | 0.1 | |
| Acesulfame-K | 0.8 | |
| Cocamidopropyl Betain (30%) | 4.5 | |
| Pluronic L35 | 1.8 | |
| Wintergreen Oil | 0.1 | A |
| Eucalyptol | 0.1 | |
| Flavor | 1 | |
| Tocopheryl Acetate | 0.05 | |
| | 100 | |

In this example, the procedure of ex. 7 is followed to obtain an opal product, suitable as toothpaste; pH = 7.0.

### Stability Test

The compositions of ex. 1 - 9 possess 36 months of shelf-life. In particular, the compositions of ex. 1 - 9
(1) did not show phase separation in 36 months of storage at room temperature as well as three months of storage at 40°C;
(2) retained 99.5%, 95% and 90% of ClO₂ precursor in one, two and three years of storage at room temperature;
(3) retained 98%, 92% and 88% of ClO₂ precursor in one, two and three years of storage at 40°C and
(4) no development of malodor, mal-taste and mal-flavor occurred in 36 months of storage in room temperature as well as three months storage in 40°C.

### Clinical Test:

A composition according to ex. 1 was clinically tested on more than 100 volunteers of different age and sex groups with halitosis problem, using a halimeter. Clinical study furnishes (1) instant extinction of bad-breath with longer than 5 hours of after-effect, (2) reduction of halitosis problem upon systematic intake of the composition over between one and three weeks, and (3) an improvement in whiteness and brightness to the tooth has been observed through "Spectroshade" study on the volunteers upon systematic use of the composition for a week.

Compositions according to ex. 7 - 9 were clinically tested on volunteers. The study furnishes an improvement in whiteness and brightness to the tooth, which has been observed through "Spectroshade" study on the volunteers upon systematic use of the composition for a week.

Compositions according to ex. 1 and 6 were tested on volunteers, after consumption of foods and drinks, which are known to cause bad breath (such as garlic enriched foods, beers, vodka, etc). The tests resulted in a positive outcome.

Furthermore, compositions according to ex. 1 and 6 were tested on volunteers suffering from Flu and shore-throat problems due to bacterial infections by means of gurgling and rinsing. While an immediate relief was noticed, it beneficially improved their health within 3 days of systematic intake.

### Size distribution:

A selection of 5 samples was provided for particle size analysis. Sample names are listed in the table below. Experimental Setup: A HELOS particle size analyzer from Sympatec GmbH was used to determine the particles size distribution of the sample provided. The HELOS analyser uses laser diffraction for the determination of particle size distributions of solids and suspensions. The system uses a laser light source including lens and measuring zones where the particles interact with laser light. The optical system converts the diffraction of the laser light in the spectrum into an image that can be detected by the photo detector. The electronic system converts the light intensity into electrical signals which are processed by software called WINDOWX, in turn this converts the data into particle size distributions.
Sample Preparation and Measurement Procedure: A background base reading of purified deionised water was taken for all samples whilst being stirred at 1200rpm by a magnetic stirrer. For samples 1-3 which were of an aqueous nature, the background water was removed from the cuvette and replaced by the test liquid. Measurements were taken a minimum of 3 times and the average value is shown in table 2. A small amount of samples 4 & 5 where placed into a dish and mixed with deionised water in order to thin the paste. This was subsequently placed in the water background until a saturation level of 15% was achieved. The stirrer was left to agitate the mixture for 5 minutes and a measurement was then taken.
Results: The results obtained are given in the table below. SMD is the average particle size and VMD is the volume mean distribution. Particles at the higher end of the particle size distribution are attributed to bubbles or to abrasive particles residing in the samples.

| **Sample Number** | **SMD (µm)** | **VMD (µm)** | **Range of Micelles (µm)** |
|---|---|---|---|
| ex.3 | 10.88 | 28.32 | 1 -8 |
| ex.1 | 25.73 | 36.34 | 8-20 |
| ex.6 | 11.09 | 37.51 | 1-20 |
| ex.9 | 5.40 | 28.56 | 1-8 |
| ex.7 | 4.52 | 17.08 | 1-8 |

The data obtained clearly show micelles in the range of 1 - 2 0 µm.
It is a surprising feature of the inventive compositions having comparatively large micelles but being transparent in appearance and stabilizing migration of lipophilic ingredients.
Without being bound to theory, it is believed this "unexpected size" (larger micelles but yet transparent) is attributed to the presence of poly-ol co-surfactants, which are fairly large molecules and present in relatively high concentration, at the interface of the inventive micelles, making it larger than normal/conventional micelles which generally form conventional micro-emulsions. A large part of these co-surfactants are believed to be a member of our inventive micelles. This attributes to their unusual stability to protect the entrapped lipophilic excipients from confronting with aqueous phase (containing oxidizing agents). In conventional micelles, "co-surfactants" are not identified as forming ingredient of the micelles, which is not the case in the inventive micelles. In conventional case, they are said to be just "adsorbed" at the surface/interface of the micelles. The excess of co-surfactants are further adsorbed at the interface of the inventive micelles (nonionic surfactant + ionic surfactants + co-surfactants) stabilizing the interface further and lowering the refractive index attributing to the transparency/opalescent appearance of our micro-emulsion (ex-1-6).

## Claims

1. A micellar composition containing a micellar phase and a continuous aqueous phase,
wherein the micellar phase comprises:
(1) one or more ionic surfactants,
(2) one or more non-ionic surfactants,
(3) one or more co-surfactants selected from the group consisting of polyols,
(4) optionally one or more lipophilic ingredients;
and wherein the continuous aqueous phase comprises:
(5) one or more oxidizing agents,
(6) one or more buffers,
(7) optionally one or more water-soluble ingredients,
(8) optionally one or more excipients;
and wherein the amount of (3) is 5 - 30 wt-% of the composition;
and wherein the ratio of (1) : (2) is in the range of 1:1.2 to 1:20;
and wherein said composition is free of ethanol; and wherein the micelles of said micellar phase have an average diameter of more than 0.1 and less than 100 micrometers, determined by laser diffraction;
and wherein the micelles of said micellar phase are mixed micelles.

2. The composition according to claim 1, wherein constituent (1) is selected from the group consisting of
i) Betaine family, Amino Betaine family, Betaine Oxide family, N-oxide family;
ii) Carboxlylate family, Sulfate family, Sarcosinate family, Sulfonate family such as, Sulfosuccinate family, Phosphate family, Acetate family and / or
iii)quaternary ammonium salts, Benzalkonium chloride, Cetylpyridinium chloride, Cetylpyridinium bromide;
and / or
wherein constituent (2) is selected from the group consisting of Sorbitan mono-, di- and tri- esters of fatty acids, their poly-ethyleneoxide (PEG) derivatives, polyethyleneglycol-polypropyleneglycol-polethylene glycol block (PEG-PPG-PEG) polymer family, polypropylene-polyethylene-polypropylene (PPG-PEG-PPG) block polymer family, family of glyceryl esters of fatty acids, family of polyglyceryl esters of fatty acids, family of glycol esters of fatty acids, family of polyglycol esters of fatty acids, fatty alcohol family, family of fatty ether alcohols, family of PPG derivatives of fatty ether alcohols, family of PEG derivatives of esters of fatty ethers and fatty acids, family of fatty amines and their PEG and PPG derivatives, family of fatty amides and their PEG derivatives;
and / or
wherein constituent (3) is selected from the group consisting of propylene glycols, glycerin, sorbitol, Polyethylene glycols, Polypropylene glycols;
and / or
wherein constituent (4) is selected from the group consisting of flavoring oils, colorants, enzymes, nutritients;
and / or
wherein constituent (5) is selected from the group consisting of Chlorine Dioxide releasing agents, Oxygen releasing agents, Chlorine releasing agents and Compounds with oxidation properties
and / or
wherein constituent (6) is selected from the group consisting of borate (Alkali metal hydroxide/Boric acid) buffer, phosphate (Alkali metal salts of phosphoric acid) buffer, carbonate/bicarbonate (alkali metal carbonates/bicarbonates) buffer, Alkali metal fluorophosphates/phosphoric acid buffer;
and / or wherein constituent (7) is selected from the group consisting of emollients, isotonic agents, and hyaluronic acid and its salts;
and / or
wherein constituent (8) is selected from the group consisting of binders, viscosity enhancers, abrasives.

3. The composition according to any of claims 1 - 2, which is free of
(1) parabenes and / or
(2) surfactants from the group of dialkyl ethers.

4. The composition according to any of claims 1 - 3, wherein said micelles have an average diameter of 0.4 - 20 micrometers, determined by laser diffraction.

5. The composition according to any of claims 1 - 4, which is selected from the group consisting of
(1) Liquids;
(2) Semi-solids; and
(3) solids.

6. The composition according to claim 5, which is selected from the group consisting of
(1) sprays, rinses;
(2) pastes, gels, creams, lotions; and
(3) powders, chewing gums, lozzanes, candies, tablets, dragees.

7. The composition according to any of claims 1 - 6 which is selected from the group consisting of
(1) compositions for oral care applications;
(2) compositions for skin care applications;
(3) compositions for hair care applications;
(4) compositions for pharmaceutical applications;
(5) compositions for household applications.

8. A method for manufacturing a composition according to any of claims 1 - 7, comprising the steps of
(a) providing a non-aqueous phase by combining in this order constituents (1), (2), optionally (4) and (3);
(b) providing an aqueous phase by combining constituents (5), (6), (7) and optionally (8) with water;
(c) combining said non - aqueous phase with said aqueous phase to obtain a micellar composition
(d) optionally combining the obtained micellar composition with further components.

9. Use of a composition according to any of claims 1 - 7
(a) for the cosmetic treatment of the teeth;
(b) for the cosmetic treatment of the skin;
(c) for the cosmetic treatment of the hair;
(d) for cleaning surfaces.

10. A Composition according to any of claims 1 - 7 for use as pharmaceutical.

11. A Composition according to any of claims 1 - 7 for use in the treatment, prevention and/or delay of progression of disorders related to halitosis, disorders of the oral cavity, disorders of the skin.

12. A micellar composition according to claim 1, wherein the micellar phase comprises one or more ionic surfactants, selected from the group of zwitterionic srufactants and anionic surfactants.

## Patentansprüche

1. Eine mizellare Zusammensetzung enthaltend eine mizellare Phase und eine kontinuierliche wässrige Phase,
wobei die mizellare Phase
(1) ein oder mehrere ionische Tenside,
(2) ein oder mehrere nicht-ionische Tenside,
(3) ein oder mehrere Cotenside, ausgewählt aus der Gruppe bestehend aus Polyolen,
(4) optional eine oder mehrere lipophile Zutaten;
umfasst,
und wobei die kontinuierliche wässrige Phase
(5) ein oder mehrere Oxidationsmittel,
(6) einen oder mehrere Puffer,
(7) optional eine oder mehrere wasserlösliche Zutaten,
(8) optional einen oder mehrere Hilfsstoffe;
umfasst,
und wobei die Menge von (3) 5 - 30 Gel.% der Zusammensetzung ist;
und wobei das Verhältnis von (1) : (2) im Bereich von 1:1.2 bis 1:20 ist;
und wobei die Zusammensetzung frei von Ethanol ist;
und wobei die Mizellen der mizellaren Phase einen Durchschnittsdurchmesser von mehr als 0.1 und von weniger als 100 Mikrometer haben, bestimmt durch Laserbeugung;
und wobei die Mizellen der mizellaren Phase gemischte Mizellen sind.

2. Die Zusammensetzung nach Anspruch 1, wobei der Bestandteil (1) aus der Gruppe bestehend aus der
(i) Betain-Familie, Aminobetain-Familie, der Betainoxid-Familie, N-Oxid-Familie;
(ii) Carboxlylat-Familie, Sulfat-Familie, Sarkosinat-Familie, Sulfonat-Familie wie Sulfosuccinat-Familie, Phosphat-Familie, Azetat-Familie und/oder
(iii) quartären Ammoniumsalzen, Benzalkoniumchlorid, Cetylpyridiniumchlorid, Cetylpyridiniumbromid;
ausgewählt ist,
und/oder
wobei der Bestandteil (2) aus der Gruppe bestehend aus
Sorbitan mono-, di- and tri- Estern von Fettsäuren, ihren Polyethylenoxid (PEG) Derivaten, Polyethylenglykol-polypropylenglykol-polethylen Glykolblock (PEG-PPG-PEG) Polymerfamilie, Polypropylenpolyethylen-polypropylen (PPG-PEG-PPG) Block Polymerfamilie, Familie von Glycerylestern von Fettsäuren, Familie von Polyglycerylestern von Fettsäuren, Familie von Glykolestern von Fettsäuren, Familie von Polyglykolestern von Fettsäuren, Fettalkohol-Familie, Familie von Fettetheralkoholen, Familie von PPG Derivaten von Fettetheralkoholen, Familie von PEG derivaten von Estern von Fettethern und Fettsäuren, Familie von Fettaminen und ihren PEG und PPG Derivaten, Familie von Fettamiden und ihren PEG Derivaten.
ausgewählt ist;
und/oder
wobei der Bestandteil (3) aus der Gruppe bestehend aus
Propylenglykolen, Glycerin, Sorbitol, Polyethylenglykolen, Polypropylenglykolen
ausgewählt ist;
und/oder
wobei der Bestandteil (4) aus der Gruppe bestehend aus
Aromaölen, Farbstoffen, Enzymen, Nährstoffen
ausgewählt ist;
und/oder
wobei der Bestandteil (5) aus der Gruppe bestehend aus
Chlordioxid-abgebenden Mitteln, Sauerstoffabgebenden Mitteln, Chlor-abgebenden Mitteln und Verbindungen mit Oxidationseigenschaften
ausgewählt ist;
und/oder
wobei der Bestandteil (6) aus der Gruppe bestehend aus
Borat (Alkalimetallhydroxide/Borsäure)-Puffern, Phosphat (Alkalimetallsalze der Phosphorsäure)-Puffern, Karbonat/Bikarbonat (Alkalimetallkarbonate /Bikarbonate)-Puffern, Alkalimetall-Fluorophosphat/Phosphorsäure-Puffern
ausgewählt ist;
und/oder
wobei der Bestandteil (7) aus der Gruppe bestehend aus
Weichmachern, isotonischen Mitteln, und Hyaluronsäure und ihren Salzen
ausgewählt ist;
und/oder
wobei der Bestandteil (8) aus der Gruppe bestehend aus
Bindemitteln, Viskositätsverbesserern, Schleifmitteln
ausgewählt ist.

3. Die Zusammensetzung nach einem der Ansprüche 1 - 2, welche frei von
(1) Parabenen und/oder
(2) Tensiden aus der Gruppe von Dialkylethern ist.

4. Die Zusammensetzung nach einem der Ansprüche 1 - 3, wobei die Mizellen einen Durchschnittsdurchmesser von 0.4 - 20 Mikrometer haben, bestimmt durch Laserbeugung.

5. Die Zusammensetzung nach einem der Ansprüche 1 - 4, welche aus der Gruppe bestehend aus
(1) Flüssigkeiten;
(2) halbfesten Stoffen; und
(3) Feststoffen
ausgewählt ist.

6. Die Zusammensetzung nach Anspruch 5, welche aus der Gruppe bestehend aus
(1) Sprays, Spülungen;
(2) Pasten, Gelen, Cremen, Lotionen, und
(3) Pudern, Kaugummis, Bonbons, Tabletten, Dragees
ausgewählt ist.

7. Die Zusammensetzung nach einem der Ansprüche 1 - 6, welche aus der Gruppe bestehend aus
(1) Zusammensetzungen für Mundpflegeanwendungen;
(2) Zusammensetzungen für Hautpflegeanwendungen;
(3) Zusammensetzungen für Haarpflegeanwendungen;
(4) Zusammensetzungen für pharmazeutische Anwendungen;
(5) Zusammensetzungen für Haushaltanwendungen;

8. Ein Verfahren zur Herstellung einer Zusammensetzung nach einem der Ansprüche 1 - 7, umfassend die Schritte
(a) Bereitstellen einer nicht-wässrigen Phase indem die Bestandteile (1), (2), und optional (4) und (3) in dieser Reihenfolge kombiniert werden;
(b) Bereitstellen einer wässrigen Phase indem die Bestandteile (5), (6), (7) und optional (8) mit Wasser kombiniert werden;
(c) Kombinieren der nicht-wässrigen Phase mit der wässrigen Phase um eine mizellare Zusammensetzung zu erhalten
(d) optional Kombinieren der erhaltenen mizellaren Zusammensetzung mit weiteren Komponenten.

9. Verwenden einer Zusammensetzung nach einem der Ansprüche 1 - 7,
(a) für die kosmetische Behandlung der Zähne;
(b) für die kosmetische Behandlung der Haut;
(c) für die kosmetische Behandlung des Haars;
(d) für die Reinigung von Oberflächen.

10. Eine Zusammensetzung nach einem der Ansprüche 1 - 7 zur Verwendung als Arzneimittel.

11. Eine Zusammensetzung nach einem der Ansprüche 1 - 7 zur Verwendung in der Behandlung, Prävention und/oder Verzögerung des Fortschreitens von Erkrankungen in Zusammenhang mit Halitosis, Erkrankungen der Mundhöhle, Erkrankungen der Haut.

12. Eine mizellare Zusammensetzung nach Anspruch 1, wobei die mizellare Phase ein oder mehrere ionische Tenside umfasst, ausgewählt aus der Gruppe von zwitterionischen Tensiden und anionischen Tensiden.

## Revendications

1. Une composition micellaire contenant une phase micellaire et une phase aqueuse continue,
la phase micellaire comprenant:
(1) un ou plusieurs tensioactifs ioniques,
(2) un ou plusieurs tensioactifs anioniques,
(3) un ou plusieurs co-tensioactifs sélectionnés du groupe consistant de polyols,
(4) optionnellement un ou plusieurs ingrédients lipophiles,
et la phase aqueuse continue comprenant
(5) un ou plusieurs agents oxydants,
(6) un ou plusieurs tampons,
(7) optionnellement un ou plusieurs ingrédients hydrosolubles,
(8) optionnellement un ou plusieurs excipients;
et la quantité de (3) étant comprise entre 5 et 30 % par poids de la composition;
et le rapport de (1) : (2) étant dans la gamme de 1:1.2 à 1:20;
et la composition étant sans éthanol;
et les micelles de ladite phase micellaire ayant un moyen diamètre de plus de 0.1 et moins de 100 micromètres, déterminé par diffraction laser;
et les micelles de ladite phase micellaire étant des micelles mélangées.

2. La composition selon la revendication 1, le composant (1) étant sélectionné du groupe consistant de
(i) la famille de bétaïne, la famille de aminobétaïne, la famille d'oxyde de bétaïne, la famille de N-oxyde;
(ii) la famille de carboxlylate, la famille de sulfate, la famille de sarcosinate, la famille de sulfonate comme, la famille de sulfosuccinate, la famille de phosphate, la famille d'acétate et/ou
(iii) des sels d'ammonium quaternaires, chlorure de benzalkonium, chlorure de cétylpyridinium, bromure de cétylpyridinium;
et/ou
le composant (2) étant sélectionné du groupe consistant de mono-, di- et triesters de sorbitan d'acides gras, leur dérivées d'oxyde de polyéthylène (PEG), la famille de polymère de bloque de glycol polyéthylèneglycol-polypropylèneglycol-poléthylène (PEG-PPG-PEG), la famille de polymère de bloque polypropylène-polyéthylène-polypropylène (PPG-PEG-PPG), la famille d'esters de glycéril d'acides gras, la famille d'esters de polyglycéril d'acides gras, la famille d'esters de glycol d'acides gras, la famille d'esters de polyglycol d'acides gras, la famille d'alcool gras, la famille d'alcools d'esters gras, la famille des dérivées PPG d'alcools d'esters gras, la famille des dérivées PEG d'esters d'éthers gras et acides gras, la famille d'amines grasses and leur dérivées PEG and PPG, la famille d'amides grasses et leur dérivées PEG;
et/ou
le composant (3) étant sélectionné du groupe consistant de propylène glycols, glycérine, sorbitol, polyéthylène glycols, polypropylène glycols;
et/ou
le composant (4) étant sélectionné du groupe consistant d'huiles aromatiques, colorants, enzymes, nutriments;
et/ou
le composant (5) étant sélectionné du groupe consistant d'agents libérant du dioxyde de chlore, agents libérant d'oxygène, agents libérant de chlore et des composants avec des caractéristiques d'oxydation;
et/ou
le composant (6) étant sélectionné du groupe consistant de tampon de borate (hydroxyde de métal alcalin/acide borique), tampon de phosphate (sels de métal alcalin d'acide phosphorique), tampon de carbonate/bicarbonate (carbonates de métal alcalin /bicarbonates), tampon de fluorophosphates de métal alcalin /acide phosphorique;
et/ou
le composant (7) étant sélectionné du groupe consistant d'émollients, agents isotoniques, et acide de hyaluronique et ses sels;
et/ou
le composant (8) étant sélectionné du groupe consistant de liants, exhausteur de viscosité, abrasives.

3. La composition selon l'une des revendications 1 - 2, étant libre de
(1) parabènes et/ou
(2) tensioactifs du groupe d'éthers de dialkyle.

4. La composition selon l'une des revendications 1 - 3, lesdites micelles ayant un diamètre moyen de 0.4 - 20 micromètres, déterminé par diffraction laser.

5. La composition selon l'une des revendications 1 - 4, sélectionnée du groupe consistant de
(1) liquides;
(2) semi-solides;
(3) solides.

6. La composition selon la revendication 5, sélectionnée du groupe consistant de
(1) sprays, rinces;
(2) pâtes, gels, crèmes, lotions; et
(3) poudres, chewing-gums, bonbons, tablettes, dragées.

7. La composition selon l'une des revendications 1 - 6, sélectionnée du groupe consistant de
(1) compositions pour des applications de soin buccale;
(2) compositions pour des applications de soin cutané;
(3) compositions pour des applications de soin des cheveux;
(4) compositions pour des applications pharmaceutiques;
(5) compositions pour des applications ménagères.

8. Un procédé pour fabriquer une composition selon l'une des revendications 1 - 7, comprenant les étapes de
(a) fournir une phase non-aqueuse en combinant dans cet ordre les composants (1), (2), optionnellement (4) et (3);
(b) fournir une phase aqueuse en combinant les composants (5), (6), (7) et optionnellement (8) avec de l'eau;
(c) combiner la phase non-aqueuse avec la phase aqueuse afin d'obtenir une composition micellaire
(d) optionnellement combiner la composition micellaire obtenue avec des autres composants.

9. Utilisation d'une composition selon l'une des revendications 1 - 7
(a) pour un traitement cosmétique des dents;
(b) pour un traitement cosmétique de la peau;
(c) pour un traitement cosmétique des cheveux;
(d) pour nettoyer des surfaces.

10. Une composition selon l'une des revendications 1 - 7 pour l'utilisation comme médicament.

11. Une composition selon l'une des revendications 1 - 7 pour l'utilisation dans le traitement, prévention et/ou le retard de la progression liée à l'halitose, des maladies de la cavité buccale, des maladies de la peau.

12. Une composition micellaire selon la revendication 1, la phase micellaire comprenant un ou plusieurs tensioactifs ioniques, sélectionnées du groupe consistant de tensioactifs zwitterioniques et tensioactifs anioniques.
